# EUROPEAN PATENT SPECIFICATION

(11) **EP 0 462 831 B1**
(45) Date of publication and mention of the grant of the patent: **28.02.1996**
(21) Application number: 91305576.0
(22) Date of filing: 19.06.1991
(51) Int. Cl.: C07D 405/12, C07D 417/12, C07D 413/12, A61K 31/415, A61K 31/425, A61K 31/42, A61K 31/40

(54) **Bicyclic pyran derivatives and their use as inhibitors of 5-lipoxygenase**
Bizyklische Pyran-Derivate und ihre Verwendung als 5-Lipoxygenasehemmer
Dérivés bicycliques et leur utilisation comme inhibiteurs de la 5-lipoxygénase

(30) Priority: 21.06.1990 EP 90401759
(43) Date of publication of application: 27.12.1991
(73) Proprietor: ZENECA LIMITED, London W1Y 6LN (GB); ZENECA Pharma S.A., F-95022 Cergy Cédex (FR)
(72) Inventor: Bruneau, Pierre Andre Raymond, F-51500 Ludes (FR); Crawley, Graham Charles, Macclesfield, Cheshire SK10 5AP (GB); Oldham, Keith, Poynton, Cheshire SK12 1UG (GB)
(74) Representative: Tait, Brian Steele

(56) References cited:
- EP-A- 0 385 662
- EP-A- 0 385 679

## Description

This invention concerns novel bicyclic heterocyclic derivatives and more particularly novel bicyclic heterocyclic derivatives which are inhibitors of the enzyme 5-lipoxygenase (hereinafter referred to as 5-LO). The invention also concerns processes for the manufacture of said bicyclic heterocyclic derivatives and novel pharmaceutical compositions containing them. Also included in the invention is the use of said bicyclic heterocyclic derivatives in the treatment of various inflammatory and/or allergic diseases in which the direct or indirect products of 5-LO catalysed oxidation of arachidonic acid are involved, and the production of new medicaments for such use.

As stated above the bicyclic heterocyclic derivatives described hereinafter are inhibitors of 5-LO, which enzyme is known to be involved in catalysing the oxidation of arachidonic acid to give rise via a cascade process to the physiologically active leukotrienes such as leukotriene B₄ (LTB₄) and the peptido-lipid leukotrienes such as leukotriene C₄ (LTC₄) and leukotriene D₄ (LTD₄) and various metabolites.

The biosynthetic relationship and physiological properties of the leukotrienes are summarised by G.W. Taylor and S.R. Clarke in Trends in Pharmacological Sciences, 1986, 7, 100-103. The leukotrienes and their metabolites have been implicated in the production and development of various inflammatory and allergic diseases such as arthritic diseases, asthma, allergic rhinitis, atopic dermatitis, psoriasis, cardiovascular and cerebrovascular disorders and inflammatory bowel disease. In addition the leukotrienes are mediators of inflammatory diseases by virtue of their ability to modulate lymphocyte and leukocyte function. Other physiologically active metabolites of arachidonic acid, such as the prostaglandins and thromboxanes, arise via the action of the enzyme cyclooxygenase on arachidonic acid.

We have now discovered that certain bicyclic heterocyclic derivatives are effective as inhibitors of the enzyme 5-LO and thus of leukotriene biosyntheses. Thus, such compounds are of value as therapeutic agents in the treatment of, for example, allergic conditions, psoriasis, asthma, cardiovascular and cerebrovascular disorders, and/or inflammatory and arthritic conditions, mediated alone or in part by one or more leukotrienes.

According to the invention there is provided a bicyclic heterocyclic derivative of the formula I (set out hereinafter) wherein Q is a 9-membered bicyclic heterocyclic moiety containing one or two nitrogen heteroatoms and optionally containing a further heteroatom selected from nitrogen, oxygen and sulphur, which heterocyclic moiety may optionally bear one or two oxo or thioxo substituents and up to four further substituents selected from halogeno, hydroxy, cyano, amino, (1-4C)alkyl, (1-4C)alkoxy, fluoro-(1-4C)alkyl, (1-4C)alkylamino, di-[(1-4C)alkyl]amino, amino-(1-4C)alkyl, (1-4C)alkylamino-(1-4C)alkyl, di-[(1-4C)alkyl]amino-(1-4C)alkyl, phenyl and phenyl-(1-4C)alkyl, and wherein said phenyl or phenyl-(1-4C)alkyl substituent may optionally bear a substituent selected from halogeno, (1-4C)alkyl and (1-4C)alkoxy;
wherein A¹ is a direct link to X¹ or is (1-3C)alkylene;
wherein X¹ is oxy, thio, sulphinyl, sulphonyl or imino;
wherein Ar is phenylene which may optionally bear one or two substituents selected from halogeno, hydroxy, amino, nitro, cyano, carbamoyl, ureido, (1-4C)alkyl, (1-4C)alkoxy, (1-4C)alkylamino, di-[(1-4C)alkyl]amino, fluoro-(1-4C)alkyl and (2-4C)alkanoylamino; or Ar is pyridylene;
wherein R¹ is (1-4C)alkyl, (3-4C)alkenyl or (3-4C)alkynyl; and wherein R² and R³ together form a group of the formula -A²-X²-A³- which, together with the carbon atom to which A² and A³ are attached, defines a ring having 5 to 7 ring atoms, wherein A² and A³, which may be the same or different, each is (1-3C)alkylene and X² is oxy, thio, sulphinyl or sulphonyl, and which ring may bear one, two or three substituents, which may be the same or different, selected from hydroxy, (1-4C)alkyl and (1-4C)alkoxy;
or wherein R¹ and R² together form a group of the formula -A²-X²-A³- which, together with the oxygen atom to which A² is attached and with the carbon atom to which A³ is attached, defines a ring having 5 to 7 ring atoms, wherein A² and A³, which may be the same or different, each is (1-3C)alkylene and X² is oxy, thio, sulphinyl or sulphonyl, and which ring may bear one, two or three (1-4C)alkyl substituents, and wherein R³ is (1-4C)alkyl, (2-4C)alkenyl or (2-4C)alkynyl;
or a pharmaceutically-acceptable salt thereof.

The chemical formulae referred to herein by Roman numerals are set out for convenience on a separate sheet hereinafter.

In this specification the generic term "alkyl" includes both straight-chain and branched-chain alkyl groups. However references to individual alkyl groups such as "propyl" are specific for the straight-chain version only and references to individual branched-chain alkyl groups such as "isopropyl" are specific for the branched-chain version only. An analogous convention applies to other generic terms.

It is to be understood that, insofar as certain of the compounds of the formula I defined above may exhibit the phenomenon of tautomerism and any formula drawing presented herein may represent only one of the possible tautomeric forms, the invention includes in its definition any tautomeric form of a compound of the formula I which possesses the property of inhibiting 5-LO and is not to be limited merely to any one tautomeric form utilised within the formulae drawings.

It is further to be understood that, insofar as certain of the compounds of formula I defined above may exist in optically active or racemic forms by virtue of one or more substituents containing an asymmetric carbon atom, the invention includes in its definition any such optically active or racemic form which possesses the property of inhibiting 5-LO. The synthesis of optically active forms may be carried out by standard techniques of organic chemistry well known in the art, for example by synthesis from optically active starting materials or by resolution of a racemic form. Similarly, inhibitory properties against 5-LO may be evaluated using the standard laboratory techniques referred to hereinafter.

Suitable values for the generic terms referred to above include those set out below.

A suitable value for Q when it is a 9-membered bicyclic heterocyclic moiety containing one or two nitrogen heteroatoms and optionally containing a further heteroatom selected from nitrogen, oxygen and sulphur is, for example, a benzo-fused heterocyclic moiety or a hydrogenated derivative thereof such as indolyl, indolinyl, isoindolyl, isoindolinyl, indolizinyl, benzimidazolyl, 2,3-dihydrobenzimidazolyl, 1H-indazolyl, 2,3-dihydro-1H-indazolyl, benzoxazolyl, 2,3-dihydrobenzoxazolyl, benzo[c]isoxazolyl, benzo[d]isoxazolyl, 2,3-dihydrobenzo[d]isoxazolyl, benzothiazolyl, 2,3-dihydrobenzothiazolyl, benzo[c]isothiazolyl, benzo[d]isothiazolyl and 2,3-dihydrobenzo[d]isothiazolyl, or, for example, a pyrido-fused heterocyclic moiety or a hydrogenated derivative thereof such as 1H-pyrrolo[2,3-b]pyridyl, 2,3-dihydro-1H-pyrrolo[2,3-b]pyridyl, 1H-pyrrolo[2,3-c]pyridyl, 2,3-dihydro-1H-pyrrolo[2,3-c]pyridyl, 1H-imidazo[4,5-b]pyridyl, 2,3-dihydro-1H-imidazo[4,5-b]pyridyl, 1H-imidazo[4,5-c]pyridyl and 2,3-dihydro-1H-imidazo[4,5-c]pyridyl. The heterocyclic moiety may be attached through any available position including from either of the two rings of the bicyclic heterocyclic moiety and including through an available nitrogen atom. The heterocyclic moiety may bear a suitable substituent such as, for example, a (1-4C)alkyl, di-[(1-4C)alkyl]amino-(1-4C)alkyl, phenyl or phenyl-(1-4C)alkyl substituent on an available nitrogen atom.

Suitable values for substituents which may be present on Q or Ar, or on a phenyl or phenyl-(1-4C)alkyl substituent on Q, include, for example:-
- for halogeno:: fluoro, chloro, bromo and iodo;
- for (1-4C)alkyl:: methyl, ethyl, propyl, isopropyl, butyl, isobutyl and sec-butyl;
- for (1-4C)alkoxy:: methoxy, ethoxy, propoxy, isopropoxy and butoxy;
- for fluoro-(1-4C)alkyl:: fluoromethyl, difluoromethyl, trifluoromethyl, 2-fluoroethyl, 2,2,2-trifluoroethyl and pentafluoroethyl;
- for (1-4C)alkylamino:: methylamino, ethylamino, propylamino and butylamino; and
- for di-[(1-4C)alkyl]amino:: dimethylamino, diethylamino and dipropylamino.

Suitable values for substituents which may be present on Q include, for example:-
- for amino-(1-4C)alkyl:: aminomethyl, 1-aminoethyl, 2-aminoethyl, 2-aminopropyl and 3-aminopropyl;
- for (1-4C)alkylamino-(1-4C)-alkyl:: methylaminomethyl, 2-methylaminoethyl, 3-methylaminopropyl, ethylaminomethyl and 2-ethylaminoethyl;
- for di-[(1-4C)alkyl]amino-(1-4C)alkyl:: dimethylaminomethyl, 2-dimethylaminoethyl, 3-dimethylaminopropyl, diethylaminomethyl and 2-diethylaminoethyl;
- for phenyl-(1-4C)alkyl:: benzyl, phenethyl and 3-phenylpropyl.

A suitable value for A¹ when it is (1-3C)alkylene is, for example, methylene, ethylene or trimethylene.

A suitable value for Ar when it is phenylene is, for example, 1,3-phenylene or 1,4-phenylene.

A suitable value for Ar when it is pyridylene is, for example, 3,5- or 2,6-pyridylene.

A suitable value for a (2-4C)alkanoylamino substituent which may be present on Ar is, for example, acetamido, propionamido or butyramido.

A suitable value for R¹ when it is (1-4C)alkyl is, for example, methyl, ethyl, propyl or butyl; when it is (3-4C)alkenyl is, for example, allyl, 2-butenyl or 3-butenyl; and when it is (3-4C)alkynyl is, for example, 2-propynyl or 2-butynyl.

When R² and R³ together form a group of the formula -A²-X²-A³- which, together with the carbon atom to which A² and A³ are attached, defines a ring having 5 to 7 ring atoms then a suitable value for A² or A³, which may be the same or different, when each is (1-3C)alkylene is, for example, methylene, ethylene or trimethylene. Suitable values for the substituents which may be present on said 5- to 7-membered ring include for example:-
- for (1-4C)alkyl:: methyl, ethyl, propyl, isopropyl, butyl and isobutyl;
- for (1-4C)alkoxy:: methoxy, ethoxy, propoxy, isopropoxy and butoxy.

When R¹ and R² together form a group of the formula -A²-X²-A³- which together with the oxygen atom to which A² is attached and with the carbon atom to which A³ is attached, defines a ring having 5 to 7 ring atoms then a suitable value for A² or A³, which may be the same or different, when each is (1-3C)alkylene is, for example, methylene, ethylene or trimethylene. Suitable values for the (1-4C)alkyl substituents which may be present on said 5- to 7-membered ring include, for example, methyl, ethyl, propyl, isopropyl and butyl.

A suitable value for R³ when it is (1-4C)alkyl is, for example, methyl, ethyl, propyl or butyl; when it is (2-4C)alkenyl is, for example, vinyl, allyl, 2-butenyl or 3-butenyl; and when it is (2-4C)alkynyl is, for example, ethynyl, 2-propynyl or 2-butynyl.

A suitable pharmaceutically-acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Particular novel compounds of the invention are, for example, bicyclic heterocyclic derivatives of the formula I wherein:-
(a) Q is a 9-membered benzo-fused heterocyclic moiety containing one nitrogen heteroatom and optionally containing a second heteroatom selected from nitrogen, oxygen and sulphur, which heterocycle may optionally bear one or two oxo or thioxo substituents and up to four substituents having the meanings defined hereinbefore for further substituents on Q; and A¹, X¹, Ar, R¹, R² and R³ have any of the meanings defined hereinbefore;
(b) Q is indolyl, indolinyl, benzimidazolyl, 2,3-dihydrobenzimidazolyl, 1H-indazolyl, 2,3-dihydro-1H-indazolyl, benzoxazolyl, 2,3-dihydrobenzoxazolyl, benzothiazolyl or 2,3-dihydrobenzothiazolyl, which may optionally bear one or two oxo or thioxo substituents and up to four substituents having the meanings defined hereinbefore for further substituents on Q; and A¹, X¹, Ar, R¹, R² and R³ have any of the meanings defined hereinbefore;
(c) Q is 2-indolyl, 3-indolyl, 5-indolyl, 6-indolyl, 2-benzimidazolyl, 5-benzimidazolyl, 6-benzimidazolyl, 1H-indazol-5-yl, 1H-indazol-6-yl, 2-benzoxazolyl, 5-benzoxazolyl, 6-benzoxazolyl, 2-benzothiazolyl, 5-benzothiazolyl or 6-benzothiazolyl, which may optionally bear one or two substituents having the meanings defined hereinbefore for further substituents on Q; and A¹, X¹, Ar, R¹, R² and R³ have any of the meanings defined hereinbefore;
(d) Q is 2-oxoindolinyl, 2,3-dioxoindolinyl, 2-oxo-2,3-dihydrobenzimidazolyl, 3-oxo-2,3-dihydro-1H-indazolyl, 2-oxo-2,3-dihydrobenzoxazolyl or 2-oxo-2,3-dihydrobenzothiazolyl, or the corresponding thioxo derivatives thereof, which may optionally bear up to four substituents having the meanings defined hereinbefore for further subsituents on Q; and A¹, X¹, Ar, R¹, R² and R³ have any of the meanings defined hereinbefore;
(e) Q is 2-oxoindolin-3-yl, 2-oxoindolin-5-yl, 2-oxoindolin-6-yl, 2,3-dioxoindolin-5-yl, 2,3-dioxoindolin-6-yl, 2-oxo-2,3-dihydrobenzimidazol-5-yl, 2-oxo-2,3-dihydrobenzimidazol-6-yl, 3-oxo-2,3-dihydro-1H-indazol-5-yl, 3-oxo-2,3-dihydro-1H-indazol-6-yl, 2-oxo-2,3-dihydrobenzoxazol-5-yl, 2-oxo-2,3-dihydrobenzoxazol-6-yl, 2-oxo-2,3-dihydrobenzothiazol-5-yl or 2-oxo-2,3-dihydrobenzothiazol-6-yl, or the corresponding thioxo derivatives thereof, which may optionally bear up to four substituents having the meanings defined hereinbefore for further substituents on Q; and A¹, X¹, Ar, R¹, R² and R³ have any of the meanings defined hereinbefore;
(f) A¹ is a direct link to X¹, and X¹ is oxy, thio, sulphinyl or sulphonyl; and Q, Ar, R¹, R² and R³ have any of the meanings defined hereinbefore;
(g) A¹ is methylene and X¹ is oxy, thio, sulphinyl or sulphonyl; and Q, Ar, R¹, R² and R³ have any of the meanings defined hereinbefore;
(h) Ar is 1,3-phenylene or 1,4-phenylene which may optionally bear one or two substituents selected from fluoro, chloro, hydroxy, amino, nitro, ureido, methyl, methoxy, methylamino, dimethylamino, trifluoromethyl and acetamido; and Q, A¹, X¹, R¹, R² and R³ have any of the meanings defined hereinbefore;
(i) Ar is 3,5-pyridylene; and Q, A¹, X¹, R¹, R² and R³ have any of the meanings defined hereinbefore;
(j) R¹ is methyl, ethyl, allyl or 2-propynyl; and Q, A¹, X¹, Ar, R² and R³ have any of the meanings defined hereinbefore;
(k) R² and R³ together form a group of the formula -A²-X²-A³- which, together with the carbon atom to which A² and A³ are attached, defines a ring having 5 to 7 ring atoms, wherein A² and A³, which may be the same or different, each is methylene, ethylene or trimethylene and X² is oxy, and which ring may bear one or two substituents selected from hydroxy, methyl, ethyl, propyl, methoxy and ethoxy; and Q, A¹, X¹, Ar and R¹ have any of the meanings defined hereinbefore; or
(l) R¹ and R² together form a group of the formula -A²-X²-A³- which, together with the oxygen atom to which A² is attached and with the carbon atom to which A³ is attached, defines a ring having 5 to 7 ring atoms, wherein A² and A³, which may be the same or different, each is methylene or ethylene and X² is oxy, and which ring may bear one, two or three substituents selected from methyl, ethyl and propyl, and R³ is methyl or ethyl; and Q, A¹, X¹ and Ar have any of the meanings defined hereinbefore;
   or a pharmaceutically-acceptable salt thereof.

A preferred compound of the invention comprises a bicyclic heterocyclic derivative of the formula I
wherein Q is indolyl, indolinyl, benzimidazolyl, 2,3-dihydrobenzimidazolyl, 1H-indazolyl, 2,3-dihydro-1H-indazolyl, benzoxazolyl, 2,3-dihydrobenzoxazolyl, benzothiazolyl or 2,3-dihydrobenzothiazolyl, which may optionally bear one or two oxo or thioxo substituents and up to four substituents selected from fluoro, chloro, bromo, hydroxy, cyano, amino, methyl, ethyl, propyl, methoxy, trifluoromethyl, 2-fluoroethyl, 2,2,2-trifluoroethyl, 2-methylaminoethyl, 2-dimethylaminoethyl, phenyl and benzyl, and wherein said phenyl or benzyl substituent may optionally bear a substituent selected from chloro, methyl and methoxy;
A¹ is a direct link to X¹ or is methylene;
X¹ is oxy, thio, sulphinyl or sulphonyl;
Ar is 1,3-phenylene or 1,4-phenylene which may optionally bear one or two substituents selected from fluoro, chloro, hydroxy, amino, nitro, ureido, methoxy, dimethylamino, trifluoromethyl and acetamido; or Ar is 3,5-pyridylene;
R¹ is methyl, ethyl, allyl or 2-propynyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl, ethyl, propyl, methoxy and ethoxy;
or R¹ and R² together form a group of the formula -A²-X²-A³- which, together with the oxygen atom to which A² is attached and with the carbon atom to which A³ is attached, defines a ring having 5 or 6 ring atoms, wherein A² is methylene, A³ is methylene or ethylene, and X² is oxy, and which ring may bear one, two or three substituents selected from methyl, ethyl and propyl, and R³ is methyl or ethyl;
or a pharmaceutically-acceptable salt thereof.

A further preferred compound of the invention comprises a bicyclic heterocyclic derivative of the formula I
wherein Q is 2-oxoindolin-5-yl, 2,3-dioxoindolin-5-yl, 2-oxo-2,3-dihydrobenzimidazol-5-yl, 2-oxo-2,3-dihydrobenzoxazol-6-yl or 2-oxo-2,3-dihydrobenzothiazol-6-yl which may optionally bear one, two or three substituents selected from fluoro, chloro, hydroxy, methyl, ethyl, propyl, 2-fluoroethyl, 2-dimethylaminoethyl, phenyl and benzyl;
A¹ is a direct link to X¹, or is methylene;
X¹ is oxy, thio, sulphinyl or sulphonyl;
Ar is 1,3-phenylene or 1,4-phenylene which may optionally bear one or two substituents selected from fluoro, hydroxy, amino, nitro, ureido, methoxy, dimethylamino, trifluoromethyl and acetamido; or
Ar is 3,5-pyridylene;
R¹ is methyl, ethyl, allyl or 2-propynyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl and ethyl;
or R¹ and R² together form a group of the formula -A²-X²-A³- which, together with the oxygen atom to which A² is attached and with the carbon atom to which A³ is attached, defines a ring having 5 ring atoms, wherein A² is methylene, A³ is methylene, and X² is oxy, and which ring may bear one, two or three substituents selected from methyl and ethyl, and R³ is methyl or ethyl;
or a pharmaceutically-acceptable salt thereof.

A further preferred compound of the invention comprises a bicyclic heterocylic derivative of the formula I
wherein Q is 2-oxoindolin-5-yl, 2-oxo-2,3-dihydrobenzimidazol-5-yl, 2-oxo-2,3-dihydrobenzoxazol-6-yl or 2-oxo-2,3-dihydrobenzothiazol-6-yl, or the corresponding N-methyl or N-ethyl derivatives thereof, which may optionally bear one or two methyl substituents;
A¹ is a direct link to X¹, or is methylene;
X¹ is oxy or thio;
Ar is 1,3-phenylene which may optionally bear one or two substituents selected from fluoro, methoxy and trifluoromethyl;
R¹ is methyl, ethyl or allyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene and X² is oxy, and which ring may bear a substituent selected from methyl, ethyl, propyl and methoxy;
or a pharmaceutically-acceptable salt thereof.

A further preferred compound of the invention comprises a bicyclic heterocyclic derivative of the formula I
wherein Q is 2-oxoindolin-5-yl, 2-oxo-2,3-dihydrobenzimidazol-5-yl, 2-oxo-2,3-dihydrobenzoxazol-6-yl or 2-oxo-2,3-dihydrobenzothiazol-6-yl, or the corresponding N-methyl or N-ethyl derivatives thereof, which may optionally bear one or two substituents selected from fluoro, hydroxy and methyl;
A¹ is a direct link to X¹, or is methylene;
X¹ is oxy or thio;
Ar is 1,3-phenylene which may optionally bear one or two substituents selected from fluoro, methoxy and trifluoromethyl;
R¹ is methyl, ethyl or allyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene and X² is oxy, and which ring may bear a substituent selected from methyl, ethyl, propyl and methoxy;
or a pharmaceutically-acceptable salt thereof.

An especially preferred compound of the invention comprises a bicyclic heterocyclic derivative of the formula I
wherein Q is 1,3,3-trimethyl-2-oxoindolin-5-yl, 1,3-dimethyl-2-oxo-2,3-dihydrobenzimidazol-5-yl or 3-methyl-2-oxo-2,3-dihydrobenzothiazol-6-yl;
A¹ is a direct link to X¹;
X¹ is thio;
Ar is 1,3-phenylene or 5-fluoro-1,3-phenylene;
R¹ is methyl; and
R² and R³ together form a group of the formula -A²-X²-A³- which, together with the carbon atom to which A² and A³ are attached, defines a ring having 6 ring atoms, wherein each of A² and A³ is ethylene and X² is oxy, and which ring may bear a methyl or ethyl substituent alpha to X²;
or a pharmaceutically-acceptable salt thereof.

A further especially preferred compound of the invention comprises a bicyclic heterocyclic derivative of the formula I wherein Q is 1,3,3-trimethyl-2-oxoindolin-5-yl, 1,3-dimethyl-2-oxo-2,3-dihydrobenzimidazol-5-yl, 3-methyl-2-oxo-2,3-dihydrobenzothiazol-6-yl or 3,3-difluoro-1-methyl-2-oxoindolin-5-yl;
A¹ is a direct link to X¹;
X¹ is thio;
Ar is 1,3-phenylene or 5-fluoro-1,3-phenylene;
R¹ is methyl; and
R² and R³ together form a group of the formula -A²-X²-A³- which, together with the carbon atom to which A² and A³ are attached, defines a ring having 6 ring atoms, wherein each of A² and A³ is ethylene and
X² is oxy, and which ring may bear a methyl or ethyl substituent alpha to X²;
or a pharmaceutically-acceptable salt thereof.

Specific especially preferred compounds of the invention include, for example, the following bicyclic heterocyclic derivatives of the formula I, or pharmaceutically-acceptable salts thereof:-
4-methoxy-4-[3-(1,3,3-trimethyl-2-oxoindolin-5-ylthio)phenyl]tetrahydropyran, 4-[3-(1,3-dimethyl-2-oxo-2,3-dihydrobenzimidazol-5-ylthio)-phenyl]-4-methoxytetrahydropyran and 4-methoxy-4-[3-(3-methyl-2-oxo-2,3-dihydrobenzothiazol-6-ylthio)phenyl]tetrahydropyran.

Further specific especially preferred compounds of the invention include, for example, the following bicyclic heterocyclic derivatives of the formula I, or pharmaceutically-acceptable salts thereof:-
4-[5-fluoro-3-(3-methyl-2-oxo-2,3-dihydrobenzothiazol-6-ylmethoxy)-phenyl]-4-methoxytetrahydropyran,
4-[5-fluoro-3-(1,3-dimethyl-2-oxo-2,3-dihydrobenzimidazol-5-ylmethoxy)phenyl]-4-methoxytetrahydropyran and
4-[5-fluoro-3-(3,3-difluoro-1-methyl-2-oxoindolin-5-ylmethoxy)phenyl]-4-methoxytetrahydropyran.

A compound of the invention comprising a bicyclic heterocyclic derivative of the formula I, or a pharmaceutically-acceptable salt thereof, may be prepared by any process known to be applicable to the preparation of structurally-related compounds. Such procedures are provided as a further feature of the invention and are illustrated by the following representative examples in which, unless otherwise stated, Q, A¹, X¹, Ar, R¹, R² and R³ have any of the meanings defined hereinbefore.
(a) The coupling, preferably in the presence of,a suitable base, of a compound of the formula Q-A¹-X¹-H with a compound of the formula II
   wherein Z is a displaceable group; provided that, when there is an amino, alkylamino or hydroxy group in Q, Ar, R² or R³, any amino, alkylamino or hydroxy group may be protected by a conventional protecting group or alternatively any such group need not be protected, whereafter any undesired protecting group in Q, Ar, R² or R³ is removed by conventional means.
   A suitable displaceable group Z is, for example, a halogeno or sulphonyloxy group, for example a chloro, bromo, iodo, methane-sulphonyloxy or toluene-p-sulphonyloxy group.
   A suitable base for the coupling reaction is, for example, an alkali or alkaline earth metal carbonate, (1-4C)alkoxide, hydroxide or hydride, for example sodium carbonate, potassium carbonate, sodium ethoxide, sodium butoxide, sodium hydroxide, potassium hydroxide, sodium hydride or potassium hydride. The coupling reaction is conveniently performed in a suitable inert solvent or diluent, for example N,N-dimethylformamide, N,N-dimethylacetamide, N-methylpyrrolidin-2-one, dimethylsulphoxide, acetone, 1,2-dimethoxyethane or tetrahydrofuran, and at a temperature in the range, for example, 10 to 150°C, conveniently at or near 100°C.
   Conveniently the reaction may be performed in the presence of a suitable catalyst, for example a metallic catalyst, for example palladium(O) or copper(I) such as tetrakis(triphenylphosphine)-palladium, cuprous chloride or cuprous bromide.
   A suitable protecting group for an amino or alkylamino group is, for example, an acyl group for example a (2-4C)alkanoyl group (especially acetyl), a (1-4C)alkoxycarbonyl group (especially methoxycarbonyl, ethoxycarbonyl or tert-butoxycarbonyl), an arylmethoxycarbonyl group (especially benzyloxycarbonyl) or an aroyl group (especially benzoyl). The deprotection conditions for the above protecting groups necessarily vary with the choice of protecting group. Thus, for example, an acyl group such as an alkanoyl or alkoxycarbonyl or an aroyl group may be removed for example, by hydrolysis with a suitable base such as an alkali metal hydroxide, for example lithium or sodium hydroxide. Alternatively an acyl group such as a tert-butoxycarbonyl group may be removed, for example, by treatment with a suitable acid such as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-charcoal.
   A suitable protecting group for a hydroxy group is, for example, an acyl group, for example a (2-4C)alkanoyl group (especially acetyl), an aroyl group (especially benzoyl) or an arylmethyl group (especially benzyl). The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-charcoal.
   The starting materials of the formula Q-A¹-X¹-H and of the formula II may be obtained by standard procedures of organic chemistry. Thus, for example, when a starting material of the formula Q-SH is required, it may be obtained by, for example, the reaction of a heterocyclic moiety of the formula Q-H as defined hereinbefore with a halosulphonylating agent such as, for example, chlorosulphonic acid, in a suitable solvent or diluent, for example dichloroethane or pyridine, and at a temperature in the range, for example 40 to 150°C, conveniently at or near 100°C. The intermediate of, for example, the formula Q-SO₂-Cl so produced may be reduced to a compound of the formula Q-SH by a conventional reducing agent such as, for example, a suitable reducing metallic salt such as a metallic halide, for example a stannous halide, conveniently stannous chloride, in a suitable solvent or diluent such as a (2-4C)alkanoic acid, for example acetic acid, and at a temperature in the range, for example, 40 to 150°C, conveniently in the range 80 to 100°C. Alternatively the reducing agent may be a suitable reducing metal, such as zinc or iron, in the presence of a strong acid, such as hydrochloric, sulphuric or phosphoric acid, and at a temperature in the range, for example 10 to 150°C, conveniently at or near 100°C.
   Conveniently intermediates of the formula II wherein Z, Ar, R¹, R² and R³ have the meanings defined hereinbefore, may be obtained by way of compounds of the formula Z-Ar-Y, wherein Z and Ar have the meanings defined hereinbefore and Y is, for example, a halogeno, formyl, alkanoyl, nitrile or alkoxycarbonyl group, as illustrated in accompanying Scheme I (set out hereinafter). Thus, for example, in the accompanying non-limiting Examples it is shown how to convert a compound of the formula Z-Ar-Y wherein Y is a halogeno group to a compound of the formula II.
   It will also be appreciated that the intermediate of the formula II may conveniently be obtained from the compound of the formula Z-Ar-Y, as defined hereinbefore, by reversing the order of introduction of the groups R² and R³ which is used in Scheme I.
(b) The coupling, preferably in the presence of a suitable base as defined hereinbefore, of a compound of the formula III with a compound of the formula Q-A¹-Z wherein Z is a displaceable group as defined hereinbefore; provided that, when there is an amino, alkylamino or hydroxy group in Q, Ar, R² or R³, any amino, alkylamino or hydroxy group may be protected by a conventional protecting group as defined hereinbefore or alternatively any such group need not be protected, whereafter any undesired protecting group in Q, Ar, R² or R³ is removed by conventional means.
   The coupling reaction is conveniently performed in a suitable inert solvent as defined hereinbefore and at a temperature in the range, for example, 10 to 150°C, conveniently at or near 100°C. The reaction may conveniently be performed in the presence of a suitable catalyst as defined hereinbefore.
   The starting materials of the formula Q-A¹-Z and of the formula III may be obtained by standard procedures of organic chemistry. The preparation of such starting materials is described within the accompanying non-limiting Examples which are provided for the purpose of illustration only. Alternatively necessary starting materials are obtainable by analogous procedures to those illustrated in accompanying Scheme II (set out hereinafter) or by modifications thereto which are within the ordinary skill of an organic chemist.
   A suitable protecting group R⁴, as employed in Scheme II, is any one of the many such groups known in the art and includes any appropriate protecting group as defined hereinbefore. Examples of such groups are given in Scheme II. The conditions for the introduction and removal of such protecting groups are described in standard textbooks of organic chemistry such as, for example, "Protective Groups in Organic Synthesis" by T W Green (J Wiley and Sons, 1981).
(c) The alkylation, preferably in the presence of a suitable base as defined hereinbefore, of a compound of the formula IV with a compound of the formula R¹-Z, wherein R¹ and Z have the meanings defined hereinbefore; provided that, when there is an amino, imino, alkylamino or hydroxy group in Q, X¹, Ar, R² or R³ any amino, imino, alkylamino or hydroxy group may be protected by a conventional protecting group or alternatively any such group need not be protected, whereafter any undesired protecting group in Q, X¹, Ar, R² or R³ is removed by conventional means.
   A suitable protecting group for an imino group is, for example, any of the protecting groups defined hereinbefore for an amino or alkylamino group.
   The tertiary alcohol starting material of the formula IV may be obtained by standard procedures of organic chemistry. Conveniently, and as illustrated in accompanying Scheme III (set out hereinafter), intermediates of the formulae Q-A¹-X¹-Ar-Y, wherein Q, A¹, X¹ and Ar have the meanings defined hereinbefore and Y is, for example, a halogeno, formyl, alkanoyl, nitrile or alkoxycarbonyl group may be utilised in the preparation of the tertiary alcohol starting material of the formula IV.
(d) For the production of those compounds of the formula I wherein R¹ and R² together form a group of the formula -A²-X²-A³- which, together with the oxygen atom to which A² is attached, defines a ring having 5 to 7 ring atoms and wherein A², X² and A³ have the meanings defined hereinbefore, and wherein R³ has the meaning defined hereinbefore; the cyclisation of a compound of the formula V upon reaction with an appropriate aldehyde or ketone, or with a hemiacetal or acetal thereof, or with a compound of the formula Z-A²-Z, wherein Z has the meaning defined hereinbefore; provided that, when there is an amino, imino, alkylamino or hydroxy group in Q, X¹ or Ar, any amino, imino, alkylamino or hydroxy group is protected by a conventional protecting group, whereafter any undesired protecting group in Q, X¹ or Ar is removed by conventional means.
   The cyclisation of a compound of the formula V with an appropriate aldehyde or ketone, or with a hemiacetal or acetal thereof, is conveniently carried out in the presence of a suitable acid. A suitable acid for the cyclisation reaction is, for example, an inorganic acid such as hydrochloric, sulphuric or phosphoric acid, or, for example, an organic acid such as p-toluenesulphonic acid or trifluoroacetic acid. The cyclisation reaction is conveniently performed in a suitable inert solvent or diluent, for example 1,2-dimethoxyethane or tetrahydrofuran. Preferably the reaction is performed using the appropriate aldehyde or ketone, or a hemiacetal or acetal derivative thereof, as both a reactant and diluent. The cyclisation is effected at a temperature in the range, for example, 20 to 150°C, conveniently at or near the boiling point of the diluent or solvent.
   The cyclisation of a compound of the formula V with a compound of the formula Z-A²-Z is conveniently carried out in the presence of a suitable base as defined hereinbefore.
   The tertiary alcohol starting material of the formula V may be obtained by standard procedures of organic chemistry. Conveniently, and as illustrated in accompanying Scheme IV (set out hereinafter), intermediates of the formula Q-A¹-X¹-Ar-Y, wherein Q, A¹, X¹, Ar and Y have the meanings defined hereinbefore, may be utilised in the preparation of the tertiary alcohol starting material of the formula V.
   A suitable protecting group R⁴, as defined hereinbefore, is employed.
(e) For the production of those compounds of the formula I wherein X¹ is a sulphinyl or sulphonyl group, wherein R² and R³ together form a group of the formula -A²-X²-A³- and X² is a sulphinyl or sulphonyl group or wherein R¹ and R² together form a group of the formula -A²-X²-A³- and X² is a sulphinyl or sulphonyl group, the oxidation of a compound of the formula I wherein X¹ is a thio group, wherein R² and R³ together form a group of the formula -A²-X²-A³- and X² is a thio group or wherein R¹ and R² together form a group of the formula -A²-X²-A³- and X² is a thio group.
   A suitable oxidising agent is, for example, any agent known in the art for the oxidation of thio to sulphinyl and/or sulphonyl, for example, hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible and with the required stoichiometric amount of oxidising agent in order to reduce the risk of over oxidation and damage to other functional groups. In general the reaction is carried out in a suitable solvent or diluent such as methylene chloride, chloroform, acetone, tetrahydrofuran or tert-butyl methyl ether and at a temperature, for example, at or near ambient temperature, that is in the range 15 to 35°C. When a compound carrying a sulphinyl group is required a milder oxidising agent may also be used, for example sodium or potassium metaperiodate, conveniently in a polar solvent such as acetic acid or ethanol. It will be appreciated that when a compound of the formula I containing a sulphonyl group is required, it may be obtained by oxidation of the corresponding sulphinyl compound as well as of the corresponding thio compound.
(f) For the production of those compounds of the formula I wherein Ar bears an alkanoylamino substituent, the acylation of a compound of the formula I wherein Ar bears an amino substituent.
   A suitable acylating agent is, for example, any agent known in the art for the acylation of amino to acylamino, for example an acyl halide, for example a (2-6C)alkanoyl chloride or bromide, in the presence of a suitable base, an alkanoic acid anhydride, for example a (2-6C)alkanoic acid anhydride, or an alkanoic acid mixed anhydride, for example the mixed anhydride formed by the reaction of an alkanoic acid and a (1-4C)alkoxycarbonyl halide, for example a (1-4C)alkoxycarbonyl chloride, in the presence of a suitable base. In general the reaction is carried out in a suitable solvent or diluent such as methylene chloride, acetone, tetrahydrofuran or tert-butyl methyl ether and at a temperature, for example, at or near ambient temperature, that is in the range 15 to 35°C. A suitable base when it is required is, for example, pyridine, 4-dimethylaminopyridine, triethylamine, ethyldiisopropylamine, N-methylmorpholine, an alkali metal carbonate, for example potassium carbonate, or an alkali metal carboxylate, for example sodium acetate.
(g) For the production of those compounds of the formula I wherein Q bears an alkyl or substituted alkyl substituent on an available nitrogen atom, or wherein Ar bears an alkoxy substituent, the alkylation of a compound of the formula I wherein Q bears a hydrogen atom on said available nitrogen atom, or wherein Ar bears a hydroxy substituent.

A suitable alkylating agent is, for example, any agent known in the art for the alkylation of an available nitrogen atom, or of hydroxy to alkoxy, for example an alkyl or substituted alkyl halide, for example a (1-6C)alkyl chloride, bromide or iodide or a substituted (1-4C)alkyl chloride, bromide or iodide, in the presence of a suitable base. A suitable base for the alkylation reaction is, for example, an alkali or alkaline earth metal carbonate, sodium hydroxide, potassium hydroxide, sodium hydride or potassium hydride. The alkylation reaction is preferably performed in a suitable inert solvent or diluent, for example N,N-dimethylformamide, dimethylsulphoxide, acetone, 1,2-dimethoxyethane or tetrahydrofuran, and at a temperature in the range, for example, 10 to 150°C, conveniently at or near ambient temperature.

When a pharmaceutically-acceptable salt of a novel compound of the formula I is required, it may be obtained, for example, by reaction of said compound with a suitable acid or base using a conventional procedure. When an optically active form of a compound of the formula I is required, it may be obtained by carrying out one of the aforesaid procedures using an optically active starting material, or by resolution of a racemic form of said compound using a conventional procedure.

Many of the intermediates defined herein are novel, for example those of the formulae IV and V and these are provided as a further feature of the invention.

As stated previously, the novel compounds of the formula I are inhibitors of the enzyme 5-LO. The effects of this inhibition may be demonstrated using one or more of the standard procedures set out below:-
a) An in vitro spectrophotometric enzyme assay system, which assesses the inhibitory properties of a test compound in a cell free system using 5-LO isolated from guinea pig neutrophils and as described by D. Aharony and R.L. Stein (J. Biol. Chem., 1986, 261(25), 11512-11519). This test provides a measure of the intrinsic inhibitory properties of a test compound against soluble 5-LO in an extracellular environment.
b) An in vitro assay system involving incubating a test compound with heparinised human blood, prior to challenge with the calcium ionophore A23187 and then indirectly measuring the inhibitory effects on 5-LO by assaying the amount of LTB₄ using specific radioimmunoassays described by Carey and Forder (F. Carey and R.A. Forder, Prostaglandins, Leukotrienes Med., 1986, 22, 57; Prostaglandins, 1984, 28, 666; Brit. J. Pharmacol. 1985, 84, 34P) which involve the use of a protein-LTB₄ conjugate produced using the procedure of Young et alia (Prostaglandins, 1983, 26(4), 605-613). The effects of a test compound on the enzyme cyclooxygenase (which is involved in the alternative metabolic pathway for arachidonic acid and gives rise to prostaglandins, thromboxanes and related metabolites) may be measured at the same time using the specific radioimmunoassay for thromboxane B₂(TxB₂) described by Carey and Forder (see above). This test provides an indication of the effects of a test compound against 5-LO and also cyclooxygenase in the presence of blood cells and proteins. It permits the selectivity of the inhibitory effect on 5-LO or cyclooxygenase to be assessed.
c) An ex vivo assay system, which is a variation of test b) above, involving administration of a test compound (usually orally as the suspension produced when a solution of the test compound in dimethylsulphoxide is added to carboxymethylcellulose), blood collection, heparinisation, challenge with A23187 and radioimmunoassay of LTB₄ and TxB₂. This test provides an indication of the bioavailability of a test compound as an inhibitor of 5-LO or cyclooxygenase.
d) An in vitro assay system involving the measurement of the inhibitory properties of a test compound against the liberation of LTC₄ and PGE₂ induced by zymosan on mouse resident peritoneal macrophages, using the procedure of Humes (J.L. Humes et alia, Biochem. Pharmacol., 1983, 32, 2319-2322) and conventional radioimmunoassay systems to measure LTC₄ and PGE₂. This test provides an indication of inhibitory effects against 5-LO and cyclooxygenase in a non-proteinaceous system.
e) An in vivo system involving the measurement of the effects of a test compound in inhibiting the inflammatory response to arachidonic acid in the rabbit skin model developed by D. Aked et alia (Brit. J. Pharmacol., 1986, 89, 431-438). This test provides an in vivo model for 5-LO inhibitors administered topically or orally.
f) An in vivo system involving measuring the effects of a test compound administered orally or introvenously on a leukotriene dependent bronchoconstriction induced by an antigen challenge in guinea pigs pre-dosed with an antihistamine (mepyramine), a β-adrenergic blocking agent (propranolol) and a cyclooxygenase inhibitor (indomethacin), using the procedure of W.H. Anderson et alia (British J. Pharmacology, 1983, 78(1), 67-574). This test provides a further in vivo test for detecting 5-LO inhibitors.
g) An in vivo system involving measuring the effects of a test compound administered orally against the liberation of LTB₄ induced by zymosan within an air pouch generated within the subcutaneous tissue of the back of male rats. The rats are anaesthetised and air pouches are formed by the injection of sterile air (20ml). A further injection of air (10ml) is similarly given after 3 days. At 6 days after the initial air injection the test compound is administered (usually orally as the suspension produced when a solution of the test compound in dimethylsulphoxide is added to hydroxypropylmethylcellulose), followed by the intrapouch injection of zymosan (1ml of a 1% suspension in physiological saline). After 3 hours the rats are killed, the air pouches are lavaged with physiological saline, and the specific radioimmunoassay described above is used to assay LTB₄ in the washings. This test provides an indication of inhibitory effects against 5-LO in an inflammatory milieu.

Although the pharmacological properties of the compounds of the formula I vary with structural changes as expected, in general compounds of the formula I possess 5-LO inhibitory effects at the following concentrations or doses in one or more of the above tests a)-f):-
- Test a):: IC₅₀ in the range, for example, 0.01-30µM;
- Test b):: IC₅₀ (LTB₄) in the range, for example, 0.01-40µM IC₅₀ (TxB₂) in the range, for example, 40-200µM;
- Test c):: oral ED₅₀(LTB₄) in the range, for example, 1-100mg/kg;
- Test d):: IC₅₀ (LTC₄) in the range, for example, 0.001-1µM, IC₅₀ (PGE₂) in the range, for example, 20-1000µM;
- Test e):: inhibition of inflammation in the range, for example, 0.3-100µg intradermally;
- Test f):: ED₅₀ in the range, for example, 0.5-10mg/kg i.v.;
- Test g):: oral ED₅₀(LTB₄) in the range, for example, 0.5-50mg/kg.

No overt toxicity or other untoward effects are present in tests c), e), f) and/or g) when compounds of the formula I are administered at several multiples of their minimum inhibitory dose or concentration.

Thus, by way of example, the compound 4-[3-(1,3-dimethyl-2-oxo-2,3-dihydrobenzimidazol-5-ylthio)phenyl]-4-methoxytetrahydropyran has an IC₅₀ of 0.05µM against LTB₄ in test b), and an oral ED₅₀ of <2mg/kg versus LTB₄ in test g). In general those compounds of the formula I which are particularly preferred have an IC₅₀ of <1µM against LTB₄ in test b), and an oral ED₅₀ of <100 mg/kg against LTB₄ in tests c) and/or g).

These compounds are examples of compounds of the invention which show selective inhibitory properties for 5-LO as opposed to cyclooxygenase, which selective properties are expected to impart improved therapeutic properties, for example, a reduction in or freedom from the gastrointestinal side-effects frequently associated with cyclooxygenase inhibitors such as indomethacin.

According to a further feature of the invention there is provided a pharmaceutical composition which comprises a bicyclic heterocyclic derivative of the formula I, or a pharmaceutically-acceptable salt thereof, in association with a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder or a liquid aerosol; for sub-lingual or buccal use, for example a tablet or capsule; or for parenteral use (including intravenous, subcutaneous, intramuscular, intravascular or infusion), for example a sterile aqueous or oily solution or suspension. In general the above compositions may be prepared in a conventional manner using conventional excipients.

The amount of active ingredient (that is a bicyclic heterocyclic derivative of the formula I, or a pharmaceutically-acceptable salt thereof) that is combined with one or more excipients to produce a single dosage form will necessarily vary depending upon the host treated and the particular route of administration. For example, a formulation intended for oral administration to humans will generally contain, for example, from 0.5 mg to 2g of active agent compounded with an appropriate and convenient amount of excipients which may vary from about 5 to about 98 percent by weight of the total composition. Dosage unit forms will generally contain about 1 mg to about 500 mg of an active ingredient.

According to a further feature of the invention there is provided a bicyclic heterocyclic derivative of the formula I, or a pharmaceutically-acceptable salt thereof, for use in a method of treatment of the human or animal body by therapy.

The invention also includes a method of treating a disease or medical condition mediated alone or in part by one or more leukotrienes which comprises administering to a warm-blooded animal requiring such treatment an effective amount of an active ingredient as defined above. The invention also provides the use of such an active ingredient in the production of a new medicament for use in a leukotriene mediated disease or medical condition.

The size of the dose for therapeutic or prophylactic purposes of a compound of the formula I will naturally vary according to the nature and severity of the conditions, the age and sex of the animal or patient and the route of administration, according to well known principles of medicine. As mentioned above, compounds of the formula I are useful in treating those allergic and inflammatory conditions which are due alone or in part to the effects of the metabolites of arachidonic acid arising by the linear (5-LO catalysed) pathway and in particular the leukotrienes, the production of which is mediated by 5-LO. As previously mentioned, such conditions include, for example, asthmatic conditions, allergic reactions, allergic rhinitis, allergic shock, psoriasis, atopic dermatitis, cardiovascular and cerebrovascular disorders of an inflammatory nature, arthritic and inflammatory joint disease, and inflammatory bowel diseases.

In using a compound of the formula I for therapeutic or prophylactic purposes it will generally be administered so that a daily dose in the range, for example, 0.5mg to 75mg per kg body weight is received, given if required in divided doses. In general lower doses will be administered when a parenteral route is employed. Thus, for example, for intravenous administration, a dose in the range, for example, 0.5mg to 30 mg per kg body weight will generally be used. Similarly, for administration by inhalation, a dose in the range, for example, 0.5 mg to 25 mg per kg body weight will be used.

Although the compounds of the formula I are primarily of value as therapeutic agents for use in warm-blooded animals (including man), they are also useful whenever it is required to inhibit the enzyme 5-LO. Thus, they are useful as pharmacological standards for use in the development of new biological tests and in the search for new pharmacological agents.

By virtue of their effects on leukotriene production, the compounds of the formula I have certain cytoprotective effects, for example they are useful in reducing or suppressing certain of the adverse gastrointestinal effects of the cyclooxygenase inhibitory non-steroidal anti-inflammatory agents (NSAIA), such as indomethacin, acetylsalicylic acid, ibuprofen, sulindac, tolmetin and piroxicam. Furthermore, co-administration of a 5-LO inhibitor of the formula I with a NSAIA can result in a reduction in the quantity of the latter agent needed to produce a therapeutic effect, thereby reducing the likelihood of adverse side-effects. According to a further feature of the invention there is provided a pharmaceutical composition which comprises a bicyclic heterocyclic derivative of the formula I, or a pharmaceutically-acceptable salt thereof as defined hereinbefore, in conjunction or admixture with a cyclooxygenase inhibitory non-steroidal anti-inflammatory agent (such as mentioned above), and a pharmaceutically-acceptable diluent or carrier.

The cytoprotective effects of the compounds of the formula I may be demonstrated, for example in a standard laboratory model which assesses protection against indomethacin-induced or ethanol-induced ulceration in the gastrointestinal tract of rats.

The compositions of the invention may in addition contain one or more therapeutic or prophylactic agents known to be of value for the disease under treatment. Thus, for example a known platelet aggregation inhibitor, hypolipidemic agent, anti-hypertensive agent, beta-adrenergic blocker or a vasodilator may usefully also be present in a pharmaceutical composition of the invention for use in treating a heart or vascular disease or condition. Similarly, by way of example, an anti-histamine, steroid (such as beclomethasone dipropionate), sodium cromoglycate, phosphodiesterase inhibitor or a beta-adrenergic stimulant may usefully also be present in a pharmaceutical composition of the invention for use in treating a pulmonary disease or condition.

The invention will now be illustrated in the following non-limiting Examples in which, unless otherwise stated:-
(i) evaporations were carried out by rotary evaporation in vacuo and work-up procedures were carried out after removal of residual solids by filtration;
(ii) operations were carried out at room temperature, that is in the range 18-25° and under an atmosphere of an inert gas such as argon;
(iii) column chromatography (by the flash procedure) and medium pressure liquid chromatography (MPLC) were performed on Merck Kieselgel silica (Art. 9385) or Merck Lichroprep RP-18 (Art. 9303) reversed-phase silica obtained from E. Meck, Darmstadt, W. Germany;
(iv) yields are given for illustration only and are not necessarily the maximum attainable;
(v) the end-products of the formula I have satisfactory microanalyses and their structures were confirmed by NMR and mass spectral techniques;
(vi) intermediates were not generally fully characterised and purity was assessed by thin layer chromatographic, infra-red (IR) or NMR analysis;
(vii) melting points are uncorrected and were determined using a Mettler SP62 automatic melting point apparatus or an oil-bath apparatus; melting points for the end-products of the formula I were determined after recrystallisation from a conventional organic solvent such as ethanol, methanol, acetone, ether or hexane, alone or in admixture; and
(viii) the following abbreviations have been used:-
   - THF: tetrahydrofuran;
   - DMSO: dimethylsulphoxide;
   - DMF: N,N-dimethylformamide;
   - DMA: N,N-dimethylacetamide.

### EXAMPLE 1

A mixture of 5-iodo-1,3-dimethyl-2,3-dihydrobenzimidazol-2-one (0.44 g), 4-(3-mercaptophenyl)-4-methoxytetrahydropyran (0.34 g), cuprous chloride (0.05 g), potassium carbonate (0.34 g) and DMF (1 ml) was stirred vigorously and heated to 140°C for 1.5 hours. The mixture was cooled to ambient temperature and partitioned between water and ethyl acetate. The organic phase was washed with water, dried (MgSO₄) and evaporated. The residue was purified by column chromatography using ethyl acetate as eluent. There was thus obtained 4-[3-(1,3-dimethyl-2-oxo-2,3-dihydrobenzimidazol-5-ylthio)phenyl]-4-methoxy-tetrahydropyran (0.285 g, 49%), m.p. 112°C.
NMR Spectrum (CDCl₃, δ values) 1.8-2.0 (m, 4H), 2.95 (s, 3H), 3.35 (s, 3H), 3.45 (s, 3H), 3.75-3.90 (m, 4H), 6.9-7.3 (m, 7H).

The 5-iodo-1,3-dimethyl-2,3-dihydrobenzimidazol-2-one used as a starting material was obtained as follows:-

A mixture of 1,1′-carbonyldiimidazole (10.7 g) and THF (100 ml) was added to a mixture of 1,2-phenylenediamine (6.48 g) and THF (150 ml) which had been cooled to approximately 5°C in an ice-water bath. The mixture was stirred at ambient temperature for 16 hours. The precipitate was filtered off giving 2,3-dihydrobenzimidazol-2-one (4.38 g, 55%), m.p. >290°C.

Sodium hydride (55% w/w dispersion in oil, 2.8 g) was added portionwise to a solution of 2,3-dihydrobenzimidazol-2-one (4.3 g) in DMF (100 ml) which had been cooled in an ice-water bath. The mixture was stirred at ambient temperature for 1.5 hours, during which period a further portion of DMF (50 ml) was added to aid the stirring of the reaction mixture. Methyl iodide (5 ml) was added and the mixture was stirred at ambient temperature for 16 hours. The mixture was evaporated and the residue was partitioned between ethyl acetate and water. The organic phase was washed with water and with brine, dried (MgSO₄) and evaporated. The residue was purified by column chromatography using a 2:1 v/v mixture of toluene and ethyl acetate as eluent. There was thus obtained 1,3-dimethyl-2,3-dihydrobenzimidazol-2-one (3.14 g, 60%), m.p. 104-106°C.

A mixture of the product so obtained (0.5 g), iodine monochloride (0.5 g) and glacial acetic acid (5 ml) was heated to 80°C for 1 hour. The mixture was partitioned between a saturated aqueous sodium sulphite solution and methylene chloride. The organic phase was washed with a saturated aqueous sodium bicarbonate solution, dried (MgSO₄) and evaporated. The residue was recrystallised from ethyl acetate. There was thus obtained 5-iodo-1,3-dimethyl-2,3-dihydrobenzimidazol-2-one (0.36 g, 41%), m.p. 142-144°C.

The 4-(3-mercaptophenyl)-4-methoxytetrahydropyran used as a starting material was obtained as follows:-

A solution of 1,3-dibromobenzene (23.8 g) in THF (120 ml) was cooled to -78°C and n-butyl-lithium (1.6M in hexane, 62.5 ml) was added dropwise. The mixture was stirred at -78°C for 30 minutes and a solution of tetrahydropyran-4-one (10 g) in THF (40 ml) was added. The resultant suspension was stirred at -78°C for 1 hour, allowed to warm to ambient temperature and then stirred for 30 minutes. The mixture was poured into brine (250 ml) and extracted with diethyl ether. The organic phase was dried (MgSO₄) and evaporated. The residue was triturated under hexane and the resultant solid (16.8 g) was filtered off.

A solution of the product so obtained in DMF (100 ml) was added dropwise to a slurry of sodium hydride (60% w/w dispersion in mineral oil; 5.25 g) in DMF (10 ml) and the mixture was stirred at ambient temperature for 90 minutes. Methyl iodide (36.5 g) was added and the mixture was stirred at ambient temperature for 16 hours. Ethanol (2 ml) and water (500 ml) was added in turn and the mixture was extracted with diethyl ether (3 x 200 ml). The combined extracts were washed with water, dried (MgSO₄) and evaporated. The residue was purified by column chromatography using increasingly polar mixtures of hexane and ethyl acetate as eluent. There was thus obtained 4-(3-bromophenyl)-4-methoxytetrahydropyran (12 g, 44%) as a gum.
NMR Spectrum (CDCl₃, δ values) 1.88-2.1 (m, 4H), 3.0 (s, 3H), 3.78-3.95 (m, 4H), 7.2-7.35 (m, 2H), 7.42 (m, 1H), 7.55 (m, 1H).

A solution of 4-(3-bromophenyl)-4-methoxytetrahydropyran (1 g) in THF (4 ml) was cooled to -80°C under an atmosphere of argon and n-butyl-lithium (1.6M in hexane, 2.4 ml) was added dropwise. The mixture was stirred at -80°C for 30 minutes, sulphur (0.12 g) was added and the mixture was stirred at -80°C for a further 30 minutes. Water (10 ml) was added and the mixture was allowed to warm to ambient temperature. The mixture was extracted with diethyl ether (10 ml). The aqueous phase was acidified to pH4 by the addition of dilute aqueous hydrochloric acid solution and extracted with diethyl ether (2 x 10 ml). The combined organic extracts were dried (MgSO₄) and evaporated. There was thus obtained the required starting material as an oil (0.5 g) which crystallised on standing and was used without further purification.

### EXAMPLE 2

The procedure described in Example 1 was repeated except that 6-iodo-3-methyl-2,3-dihydrobenzothiazol-2-one was used in place of 5-iodo-1,3-dimethyl-2,3-dihydrobenzimidazol-2-one. There was thus obtained 4-methoxy-4-[3-(3-methyl-2-oxo-2,3-dihydrobenzothiazol-6-ylthio)phenyl]tetrahydropyran in 69% yield, m.p. 113-114°C.
NMR Spectrum (CDCl₃, δ values) 1.85-2.05 (m, 4H), 3.0 (s, 3H), 3.45 (s, 3H), 3.75-3.9 (m, 4H), 7.0-7.5 (m, 7H).

The 6-iodo-3-methyl-2,3-dihydrobenzothiazol-2-one used as a starting material was obtained as follows:-

The procedures described in the portion of Example 1 which are concerned with the preparation of 5-iodo-1,3-dimethyl-2,3-dihydrobenzimidazol-2-one were repeated except that 2-aminophenylthiol was used in place of 1,2-phenylenediamine. There were thus obtained in turn:-
2,3-dihydrobenzothiazol-2-one in 55% yield, m.p. 133-134°C;
3-methyl-2,3-dihydrobenzothiazol-2-one in 77% yield, m.p. 74-75°C; and
6-iodo-3-methyl-2,3-dihydrobenzothiazol-2-one in 37% yield, m.p. 130-131°C.

### EXAMPLE 3

The procedure described in Example 1 was repeated except that 6-iodo-3-methyl-2,3-dihydrobenzoxazol-2-one was used in place of 5-iodo-1,3-dimethyl-2,3-dihydrobenzimidazol-2-one. There was thus obtained 4-methoxy-4-[3-(3-methyl-2-oxo-2,3-dihydrobenzoxazol-6-ylthio)phenyl]tetrahydropyran in 71% yield, m.p. 90-92°C.
NMR Spectrum (CDCl₃, δ values) 1.85-2.05 (m, 4H), 2.95 (s, 3H), 3.4 (s, 3H), 3.75-3.90 (m, 4H), 6.9-7.35 (m, 7H)

The 6-iodo-3-methyl-2,3-dihydrobenzoxazol-2-one used as a starting material was obtained as follows:-

A mixture of 1,1′-carbonyldiimidazole (14.6 g), 2-aminophenol (6.54 g) and THF (200 ml) was heated to reflux for 2 hours. The mixture was evaporated and the residue was partitioned between ethyl acetate and water. The organic phase was washed with 2N aqueous hydrochloric acid, water and brine, dried (MgSO₄) and evaporated. The residue was purified by column chromatography using a 2:1 v/v mixture of toluene and ethyl acetate as eluent. There was thus obtained 2,3-dihydrobenzoxazol-2-one (6.96 g, 86%), m.p. 136-138°C.

The procedures described in the portion of Example 1 which are concerned with the preparation of 5-iodo-1,3-dimethyl-2,3-dihydrobenzimidazol-2-one were repeated except that 2,3-dihydrobenzoxazol-2-one was used in place of 2,3-dihydrobenzimidazol-2-one. There were thus obtained 3-methyl-2,3-dihydrobenzoxazol-2-one in 63% yield, m.p. 80-82°C; and 6-iodo-3-methyl-2,3-dihydrobenzoxazol-2-one in 36% yield, m.p. 184-185°C.

### EXAMPLE 4

A solution of potassium peroxymonosulphate (0.32 g) in water (2 ml) was added to a solution of 4-[3-(1,3-dimethyl-2-oxo-2,3-dihydrobenzimidazol-5-ylthio)phenyl]-4-methoxytetiahydropyran (0.125 g) in ethanol (2 ml) and the resultant mixture was stirred at ambient temperature for 66 hours. The mixture was partitioned between methylene chloride and water. The organic phase was washed with water, dried (MgSO₄) and evaporated. The residue was purified by column chromatography using ethyl acetate as eluent. There was thus obtained 4-[3-(1,3-dimethyl-2-oxo-2,3-dihydrobenzimidazol-5-ylsulphonyl)phenyl]-4-methoxytetrahydropyran (0.094 g, 69%), m.p. 214-215°C.
NMR Spectrum (CDCl₃, δ values) 1.9-2.05 (m, 4H), 2.95 (s, 3H), 3.45 (2 s's, 6H), 3.8-3.9 (m, 4H), 7.0-8.0 (m, 7H).

### EXAMPLE 5

Using a similar procedure to that described in Example 4, 4-methoxy-4-[3-(3-methyl-2-oxo-2,3-dihydrobenzothiazol-6-ylthio)-phenyl]tetrahydropyran was oxidised with potassium peroxymonosulphate to give 4-methoxy-4-[3-(3-methyl-2-oxo-2,3-dihydrobenzothiazol-6-ylsulphonyl)phenyl]tetrahydropyran in 75% yield, m.p. 174-176°C.
NMR Spectrum (CDCl₃, δ values) 1.85-2.05 (m, 4H), 2.95 (s, 3H), 3.5 (s, 3H), 3.8-3.9 (m, 4H), 7.1-8.05 (m, 7H).

### EXAMPLE 6

Using a similar procedure to that described in Example 4, 4-methoxy-4-[3-(3-methyl-2-oxo-2,3-dihydrobenzoxazol-6-ylthio)-phenyl]tetrahydropyran was oxidised with potassium peroxymonosulphate to give 4-methoxy-4-[3-(3-methyl-2-oxo-2,3-dihydrobenzoxazol-6-ylsulphonyl)phenyl]tetrahydropyran in 73% yield, m.p. 163-165°C.
NMR Spectrum (CDCl₃, δ values) 1.9-2.1 (m, 4H), 2.95 (s, 3H), 3.4 (s, 3H), 3.8-3.9 (m, 4H), 7.1-8.0 (m, 7H).

### EXAMPLE 7

A mixture of 6-bromomethyl-3-methyl-2,3-dihydrobenzothiazol-2-one (0.36 g), 4-(5-fluoro-3-hydroxyphenyl)-4-methoxytetrahydropyran (0.3 g), potassium carbonate (0.276 g) and DMF (5 ml) was stirred at ambient temperature for 16 hours. The mixture was poured onto a mixture of ice and water (10 ml) and extracted with ethyl acetate. The organic phase was washed with brine, dried (MgSO₄) and evaporated. The residue was triturated in a mixture of diethyl ether and hexane. There was thus obtained 4-[5-fluoro-3-(3-methyl-2-oxo-2,3-dihydrobenzothiazol-6-ylmethoxy)phenyl]-4-methoxytetrahydropyran (0.414 g, 77%), m.p. 104-106°C.
NMR Spectrum (CDCl₃, δ values) 1.9-2.0 (m, 4H), 3.0 (s, 3H), 3.5 (s, 3H), 3.8-3.9 (m, 4H), 5.05 (s, 2H), 6.6-6.8 (m, 3H), 7.0-7.1 (m, 1H), 7.4 (m, 1H), 7.5 (d, 1H).

The 6-bromomethyl-3-methyl-2,3-dihydrobenzothiazol-2-one used as a starting material was obtained as follows:-

1,1′-Carbonyldiimidazole (2 g) was added to a suspension of 2-amino-5-methylphenylthiol (1.39 g; J. Chem. Soc. (C), 1969, 2148) in THF (40 ml) which had been cooled in an ice-bath. The mixture was stirred for 16 hours and allowed to warm to ambient temperature. The mixture was evaporated. The residue was triturated in a mixture of diethyl ether and water to give 6-methyl-2,3-dihydrobenzothiazol-2-one (0.57 g, 36%), m.p. 163-166°C. The mother liquors were evaporated and the resultant residue was partitioned between ethyl acetate and water. The organic phase was washed with brine, dried (MgSO₄) and evaporated. The residue was triturated in diethyl ether. There was thus obtained a second crop of the benzothiazol-2-one (0.4 g, 24%).

Sodium hydride (55% dispersion in oil, 0.26 g) was added to a mixture of the product so obtained (0.95 g) and DMF (10 ml) which had been cooled in an ice-water bath. The mixture was stirred at 0°C for 1.5 hours and then cooled to -20°C. Methyl iodide (0.85 g) in DMF (4 ml) was added dropwise and the mixture was stirred for 3 hours and allowed to warm to 5°C. The mixture was partitioned between cold dilute hydrochloric acid and ethyl acetate. The organic phase was washed with water and brine, dried (MgSO₄) and evaporated. The residue was purified by column chromatography using a 19:1 v/v mixture of toluene and ethyl acetate as eluent. There was thus obtained 3,6-dimethyl-2,3-dihydrobenzothiazol-2-one (0.85 g, 83%), m.p. 70-71°C.

A mixture of the product so obtained (0.83 g), N-bromosuccinimide (0.87 g), azobisisobutyronitrile (0.01 g) and carbon tetrachloride (20 ml) was stirred and heated to reflux for 2 hours. The mixture was cooled to ambient temperature and partitioned between methylene chloride and water. The organic phase was dried (MgSO₄) and evaporated. There was thus obtained 6-bromomethyl-3-methyl-2,3-dihydrobenzothiazol-2-one (0.8 g, 67%), m.p. 146-149°C.
NMR Spectrum (CDCl₃, δ values) 3.45 (s, 3H), 4.5 (s, 2H), 7.0 (d, 1H), 7.3-7.4 (m, 1H), 7.5 (d, 1H).

The 4-(5-fluoro-3-hydroxyphenyl)-4-methoxytetrahydropyran used as a starting material was obtained as follows:-

Sodium hydride (50% w/w dispersion in mineral oil, 12.4 g) was added portionwise to a mixture of benzyl alcohol (26.7 ml) and DMA (500 ml) and the mixture was stirred at ambient temperature for 1 hour. 1-Bromo-3,5-difluorobenzene (50 g) was added carefully to control the vigour of the ensuing exothermic reaction. The mixture was stirred at ambient temperature for 2 hours and the solvent was evaporated. The residue was partitioned between methylene chloride and water and the organic phase was washed with water (4 x 50 ml), dried (MgSO₄) and evaporated. The residue was purified by distillation to give 3-benzyloxy-1-bromo-5-fluorobenzene (41.8 g, 57%), as a colourless liquid (b.p. 124-130°C at 0.3 mm Hg).

A solution of a portion (9.75 g) of this product in THF (150 ml) was cooled to -75°C and n-butyl-lithium (1.6M in hexane, 22 ml) was added dropwise. The mixture was stirred at -75°C for 1 hour and a solution of tetrahydropyran-4-one (3.47 g) in THF (10 ml) was added dropwise. The mixture was stirred at -75°C for 1 hour and then allowed to warm to 0°C. A saturated aqueous ammonium chloride solution (50 ml) was added and the organic phase was separated, dried (MgSO₄) and evaporated. The residue was purified by column chromatography using a 1:1 v/v mixture of toluene and ethyl acetate as eluent. There was thus obtained 4-(3-benzyloxy-5-fluorophenyl)-4-hydroxytetrahydropyran (7.4 g, 71%) as an oil.

After appropriate repetition of the above-mentioned reaction the product so obtained (12.1 g) was dissolved in THF (150 ml) and sodium hydride (50% w/w dispersion in mineral oil, 2.11 g) was added portionwise. The mixture was stirred at ambient temperature for 1 hour, cooled in an ice-bath and methyl iodide (3.75 ml) was added dropwise. The mixture was stirred at ambient temperature for 18 hours, 2N aqueous hydrochloric acid (3 drops) was added and the organic solvent was evaporated. The residue was partitioned between ethyl acetate and water. The organic phase was separated, washed with water and with brine, dried (MgSO₄) and evaporated. There was thus obtained 4-(3-benzyloxy-5-fluorophenyl)-4-methoxytetrahydropyran (12.5 g, 99%), as a pale yellow oil which was used without further purification.

A solution of the product so obtained in ethanol (100 ml) was hydrogenated in the presence of 10% palladium-on-charcoal catalyst for 3 hours. The mixture was filtered and the filtrate was evaporated. There was thus obtained 4-(5-fluoro-3-hydroxyphenyl)-4-methoxytetrahydropyran (7.7 g, 86%), m.p. 123-124°C.

### EXAMPLE 8

The procedure described in Example 7 was repeated except that 6-bromomethyl-3-methyl-2,3-dihydrobenzoxazol-2-one was used in place of 6-bromomethyl-3-methyl-2,3-dihydrobenzothiazol-2-one. There was thus obtained 4-[5-fluoro-3-(3-methyl-2-oxo-2,3-dihydrobenzoxazol-6-ylmethoxy)phenyl]-4-methoxytetrahydropyran in 80% yield, m.p. 139-141°C.
NMR Spectrum (CDCl₃, δ values) 1.9-2.0 (m, 4H), 3.0 (s, 3H), 3.4 (s, 3H), 3.8-3.9 (m, 4H), 5.1 (s, 2H), 6.6-6.8 (m, 3H), 7.0 (m, 1H), 7.2-7.3 (m, 2H).

The 6-bromomethyl-3-methyl-2,3-dihydrobenzoxazol-2-one used as a starting material was obtained as follows:-

The procedures described in the second and third paragraphs of the portion of Example 7 which is concerned with the preparation of 6-bromomethyl-3-methyl-2,3-dihydrobenzothiazol-2-one were repeated except that 6-methyl-2,3-dihydrobenzoxazol-2-one (J. Het. Chem., 1982, 19, 1545) was used in place of 6-methyl-2,3-dihydrobenzothiazol-2-one. There were thus obtained in turn:- 3,6-dimethyl-2-oxo-2,3-dihydrobenzoxazol-2-one in 80% yield, m.p. 108-110°C; and
6-bromomethyl-3-methyl-2,3-dihydrobenzoxazol-2-one in 80% yield, m.p. 145-150°C, NMR Spectrum (CDCl₃, δ values) 3.4 (s, 3H), 4.55 (s, 2H), 6.9 (d, 1H), 7.2-7.3 (m, 2H).

### EXAMPLE 9

The procedure described in Example 7 was repeated except that 6-bromo-5-bromomethyl-1,3-dimethyl-2,3-dihydrobenzimidazol-2-one was used in place of 6-bromomethyl-3-methyl-2,3-dihydrobenzothiazol-2-one. There was thus obtained 4-[3-(6-bromo-1,3-dimethyl-2-oxo-2,3-dihydrobenzimidazol-5-ylmethoxy)-5-fluorophenyl]-4-methoxytetrahydropyran in 50% yield, m.p. 161-162°C.
NMR Spectrum (CDCl₃, δ values) 1.9-2.1 (m, 4H), 3.0 (s, 3H), 3.4 (s, 6H), 3.8-3.9 (m, 4H), 5.2 (s, 2H), 6.6-6.9 (m, 3H), 7.15 (s, 1H), 7.2 (s, 1H).

The 6-bromo-5-bromomethyl-1,3-dimethyl-2,3-dihydrobenzimidazol-2-one used as a starting material was obtained as follows:-

The procedures described in the first and second paragraphs of the portion of Example 7 which is concerned with the preparation of 6-bromomethyl-3-methyl-2,3-dihydrobenzothiazol-2-one were repeated except that 3,4-diaminotoluene was used in place of 2-amino-5-methylphenylthiol. There were thus obtained in turn:- 5-methyl-2,3-dihydrobenzimidazol-2-one in 75% yield, m.p. >250°C; and 1,3,5-trimethyl-2,3-dihydrobenzimidazol-2-one in 77% yield, m.p. 102°C.

A mixture of the product so obtained (1.4 g), N-bromosuccinimide (1.5 g), azobisisobutyronitrile (5 mg) and carbon tetrachloride (30 ml) was stirred and heated to reflux. A second portion (5 mg) of azobisisobutyronitrile was added and the mixture was maintained at reflux for 1.5 hours. The mixture was cooled to ambient temperature and partitioned between methylene chloride and water. The organic phase was washed with water, dried (MgSO₄) and evaporated. The solid residue was washed with diethyl ether and dried. There was thus obtained 6-bromo-1,3,5-trimethyl-2,3-dihydrobenzimidazol-2-one (0.4 g, 69%), m.p. 132-137°C.

The product so obtained was treated with N-bromosuccinimide using the procedure described immediately above. There was thus obtained 6-bromo-5-bromomethyl-1,3-dimethyl-2,3-dihydrobenzimidazol-2-one in 84% yield, as a solid.
NMR Spectrum (CDCl₃, δ values) 3.4 (2 s's, 6H), 4.7 (s, 2H), 7.05 (s, 1H), 7.26 (s, 1H).

### EXAMPLE 10

A mixture of 4-[3-(6-bromo-1,3-dimethyl-2-oxo-2,3-dihydrobenzimidazol-5-ylmethoxy)-5-fluorophenyl]-4-methoxytetrahydropyran (0.19 g), sodium formate (0.06 g), tetrakis(triphenylphosphine)-palladium (0.047 g) and DMF (2 ml) was heated to 100°C for 2 hours. Equivalent amounts of sodium formate and the palladium catalyst were added portionwise over the following 4 hours as the mixture was maintained at a temperature of 100°C. The mixture was cooled to ambient temperature and partitioned between ethyl acetate and water. The organic phase was washed with water and with brine, dried (MgSO₄) and evaporated. The residue was purified by column chromatography using a 5:3 v/v mixture of toluene and ethyl acetate as eluent. The product so obtained was triturated in diethyl ether. The solid so obtained was filtered off and dried. There was thus obtained 4-[5-fluoro-3-(1,3-dimethyl-2-oxo-2,3-dihydrobenzimidazol-5-ylmethoxy)phenyl]-4-methoxytetrahydropyran (0.09 g, 57%), m.p. 136-137°C.
NMR Spectrum (CDCl₃, δ values) 1.9-2.0 (m, 4H), 3.0 (s, 3H), 3.45 (2 s's, 6H), 3.8-3.9 (m, 4H), 5.1 (s, 2H), 6.6-6.8 (m, 3H), 7.0-7.2 (m, 3H).

### EXAMPLE 11

The procedure described in Example 1 was repeated except that 5-iodo-1,3,3-trimethylindolin-2-one was used in place of 5-iodo-1,3-dimethyl-2,3-dihydrobenzimidazol-2-one. The crude product so obtained was purified by column chromatography using increasingly polar mixtures of hexane and ethyl acetate as eluent. The product so obtained was further purified by column chromatography on reverse phase silica using decreasingly polar mixtures of methanol and water as eluent. There was thus obtained 4-methoxy-4-[3-(1,3,3-trimethyl-2-oxoindolin-5-ylthio)phenyl]tetrahydropyran in 23% yield, m.p. 132-134°C.
NMR Spectrum (CDCl₃, δ values) 1.35 (s, 6H), 1.85-2.05 (m, 4H), 2.95 (s, 3H), 3.25 (s, 3H), 3.75-3.85 (m, 4H), 6.85 (d, 1H), 7.1 (m, 1H), 7.18-7.3 (m, 4H), 7.4 (m, 1H).

The 5-iodo-1,3,3-trimethylindolin-2-one used as a starting material was obtained as follows:-
The procedure described in the last paragraph of the portion of Example 1 which is concerned with the preparation of 5-iodo-1,3-dimethyl-2,3-dihydrobenzimidazol-2-one was repeated except that 1,3,3-trimethylindolin-2-one (J. Amer. Chem. Soc., 1934, 56, 1797) was used as the starting material. There was thus obtained the required starting material in 76% yield, m.p. 153-154°C.
NMR Spectrum (CDCl₃, δ values) 1.45 (s, 6H), 3.2 (s, 3H), 6.54 (d, 1H), 7.48 (d, 1H), 7.6 (d of d's, 1H).

### EXAMPLE 12

Using a similar procedure to that described in Example 4, 4-methoxy-4-[3-(1,3,3-trimethyl-2-oxoindolin-5-ylthio)phenyl]-tetrahydropyran was oxidised with potassium peroxymonosulphate to give 4-methoxy-4-[3-(1,3,3-trimethyl-2-oxoindolin-5-ylsulphonyl)phenyl]-tetrahydropyran in 80% yield, m.p. 164-166°C.
NMR Spectrum (CDCl₃, δ values) 1.48 (s, 6H), 1.88-2.10 (m, 4H), 2.95 (s, 3H), 3.25 (s, 3H), 3.8-3.9 (m, 4H), 6.9 (d, 1H), 7.55 (m, 1H), 7.6 (m, 1H), 7.75 (m, 1H), 7.85 (m, 1H), 7.90 (m, 1H), 7.95 (m, 1H).

### EXAMPLE 13

The procedure described in Example 7 was repeated except that 5-bromomethyl-1,3,3-trimethylindolin-2-one was used in place of 6-bromomethyl-3-methyl-2,3-dihydrobenzothiazol-2-one. The crude product so obtained was purified by column chromatography on alumina using a 4:1 v/v mixture of methylene chloride and acetone as eluent. There was thus obtained 4-[5-fluoro-3-(1,3,3-trimethyl-2-oxoindolin-5-ylmethoxy)phenyl]-4-methoxytetrahydropyran in 17% yield, m.p. 124-126°C.

The 5-bromomethyl-1,3,3-trimethylindolin-2-one used as a starting material was obtained as follows:-

A mixture of 1,3,3,5-tetramethylindolin-2-one (0.378 g; Chem. Abs., 1971, 77, 116019g), N-bromosuccinimide (0.484 g), benzoyl peroxide (0.01 g) and carbon tetrachloride (10 ml) was stirred and irradiated with the light from a 250 Watt lamp. The mixture was heated to 40°C for 75 minutes. The mixture was cooled to ambient temperature, petroleum ether (b.p. 40-60°C) was added and the mixture was filtered. The filtrate was evaporated to give the required starting material as an oil (0.65 g, 70%) which was used without further purification.

### EXAMPLE 14

The procedure described in Example 7 was repeated except that 5-bromomethyl-1-methylindolin-2,3-dione was used in place of 6-bromomethyl-3-methyl-2,3-dihydrobenzothiazol-2-one. The crude product was purified by column chromatography using increasingly polar mixtures of methylene chloride and diethyl ether as eluent. There was thus obtained 4-[5-fluoro-3-(1-methyl-2,3-dioxoindolin-5-ylmethoxy)-phenyl]-4-methoxytetrahydropyran in 75% yield, m.p. 138-140°C.

The 5-bromomethyl-1-methylindolin-2,3-dione used as a starting material was obtained from 1,5-dimethylindolin-2,3-dione (Chem. Abs., 1983, 99, 105082a) using a similar procedure to that described in the portion of Example 13 which is concerned with the preparation of starting materials except that the reaction mixture was heated to reflux for 1 hour. There was thus obtained the required starting material in 58% yield as a solid which was used without further purification.

### EXAMPLE 15

Water (approximately 4 ml) was added dropwise to a solution of 4-[5-fluoro-3-(1-methyl-2,3-dioxoindolin-5-ylmethoxy)phenyl]-4-methoxytetrahydropyran (0.16 g) in 1,4-dioxane (3 ml) until a solid began to be precipitated. The mixture was heated to reflux and sodium dithionite (0.143 g) was added in four portions over 15 minutes and after each addition water (1 ml) was added to wash all of the dithionite into the reaction mixture. The reaction mixture was cooled and poured into water. The mixture was extracted with diethyl ether and then with ethyl acetate. The combined extracts were washed with water and brine, dried (MgSO₄) and evaporated. The residue was purified by column chromatography using a 19:1 v/v mixture of methylene chloride and methanol as eluent. There was thus obtained 4-[5-fluoro-3-(3-hydroxy-1-methyl-2-oxoindolin-5-ylmethoxy)phenyl]-4-methoxytetrahydropyran (0.126 g, 79%), m.p. 134-139°C (recrystallised from diethyl ether).

### EXAMPLE 16

Using a similar procedure to that described in Example 1, 5-iodo-1,3-dimethyl-2,3-dihydrobenzimidazol-2-one was reacted with 4-(5-fluoro-3-mercaptophenyl)-4-methoxytetrahydropyran to give 4-[3-(1,3-dimethyl-2-oxo-2,3-dihydrobenzimidazol-5-ylthio)-5-fluoro-phenyl]-4-methoxytetrahydropyran in 60% yield, m.p. 128-130°C.

The 4-(5-fluoro-3-mercaptophenyl)-4-methoxytetrahydropyran, used as a starting material, is described in European Patent Application No. 0420511 (Example 4 thereof).

### EXAMPLE 17

Diethylaminosulphur trifluoride (0.082 ml) was added dropwise to a stirred solution of 4-[5-fluoro-3-(1-methyl-2,3-dioxoindol-5-ylmethoxy)phenyl]-4-methoxytetrahydropyran (0.1 g) in a mixture of methylene chloride (2.5 ml) and fluorotrichloromethane (2.5 ml) which had been cooled to 0°C. The mixture was allowed to warm to ambient temperature and stirred at this temperature for 16 hours. The mixture was evaporated and the residue was purified by column chromatography using increasingly polar mixtures of methylene chloride and diethyl ether as eluent. There was thus obtained 4-[5-fluoro-3-(3,3-difluoro-1-methyl-2-oxoindolin-5-ylmethoxy)phenyl]-4-methoxytetrahydropyran (0.03 g, 29%), as a foam which crystallised on trituration under diethyl ether, m.p. 151°C.

### EXAMPLE 18

The following illustrate representative pharmaceutical dosage forms containing the compound of formula I, or a pharmaceutically-acceptable salt thereof (hereafter compound X), for therapeutic or prophylactic use in humans:
(a)

| Tablet I | mg/tablet |
|---|---|
| Compound X | 100 |
| Lactose Ph.Eur | 182.75 |
| Croscarmellose sodium | 12.0 |
| Maize starch paste (5% w/v paste) | 2.25 |
| Magnesium stearate | 3.0 |

(b)

| Tablet II | mg/tablet |
|---|---|
| Compound X | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |

(c)

| Tablet III | mg/tablet |
|---|---|
| Compound X | 1.0 |
| Lactose Ph.Eur | 93.25 |
| Croscarmellose sodium | 4.0 |
| Maize starch paste (5% w/v paste) | 0.75 |
| Magnesium stearate | 1.0 |

(d)

| Capsule | mg/capsule |
|---|---|
| Compound X | 10 |
| Lactose Ph.Eur | 488.5 |
| Magnesium stearate | 1.5 |

(e)

| Injection I | (50 mg/ml) |
|---|---|
| Compound X | 5.0% w/v |
| 1M Sodium hydroxide solution 0.1M Hydrochloric acid (to adjust pH to 7.6) | 15.0% v/v |
| Polyethylene glycol 400 | 4.5% w/v |
| Water for injection to 100% | |

(f)

| Injection II | (10 mg/ml) |
|---|---|
| Compound X | 1.0% w/v |
| Sodium phosphate BP | 3.6% w/v |
| 0.1M Sodium hydroxide solution | 15.0% v/v |
| Water for injection to 100% | |

(g)

| Injection III | (1mg/ml,buffered to pH6) |
|---|---|
| Compound X | 0.1% w/v |
| Sodium phosphate BP | 2.26% w/v |
| Citric acid | 0.38% w/v |
| Polyethylene glycol 400 | 3.5% w/v |
| Water for injection to 100% | |

(h)

| Aerosol I | mg/ml |
|---|---|
| Compound X | 10.0 |
| Sorbitan trioleate | 13.5 |
| Trichlorofluoromethane | 910.0 |
| Dichlorodifluoromethane | 490.0 |

(i)

| Aerosol II | mg/ml |
|---|---|
| Compound X | 0.2 |
| Sorbitan trioleate | 0.27 |
| Trichlorofluoromethane | 70.0 |
| Dichlorodifluoromethane | 280.0 |
| Dichlorotetrafluoroethane | 1094.0 |

(j)

| Aerosol III | mg/ml |
|---|---|
| Compound X | 2.5 |
| Sorbitan trioleate | 3.38 |
| Trichlorofluoromethane | 67.5 |
| Dichlorodifluoromethane | 1086.0 |
| Dichlorotetrafluoroethane | 191.6 |

(k)

| Aerosol IV | mg/ml |
|---|---|
| Compound X | 2.5 |
| Soya lecithin | 2.7 |
| Trichlorofluoromethane | 67.5 |
| Dichlorodifluoromethane | 1086.0 |
| Dichlorotetrafluoroethane | 191.6 |

### Note

The above formulations may be obtained by conventional procedures well known in the pharmaceutical art. The tablets (a)-(c) may be enteric coated by conventional means, for example to provide a coating of cellulose acetate phthalate. The aerosol formulations (h)-(k) may be used in conjunction with standard, metered dose aerosol dispensers, and the suspending agents sorbitan trioleate and soya lecithin may be replaced by an alternative suspending agent such as sorbitan monooleate, sorbitan sesquioleate, polysorbate 80, polyglycerol oleate or oleic acid.

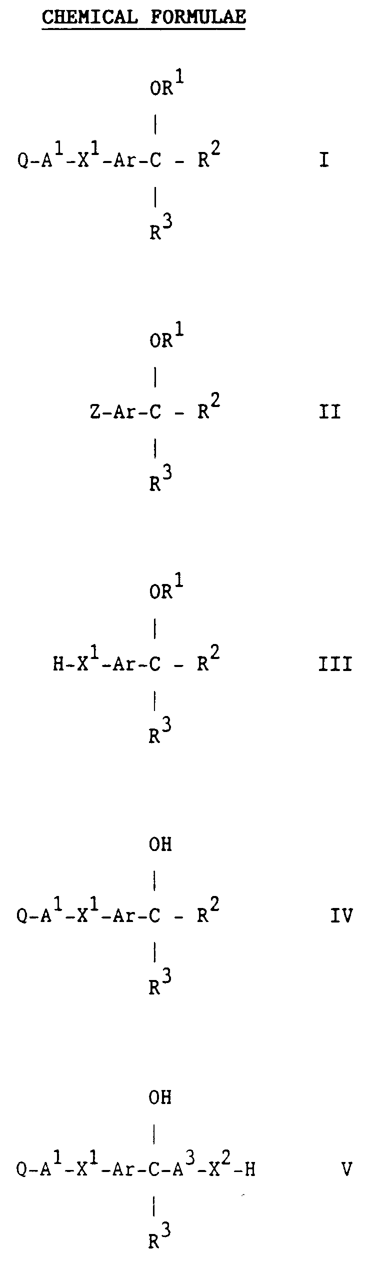

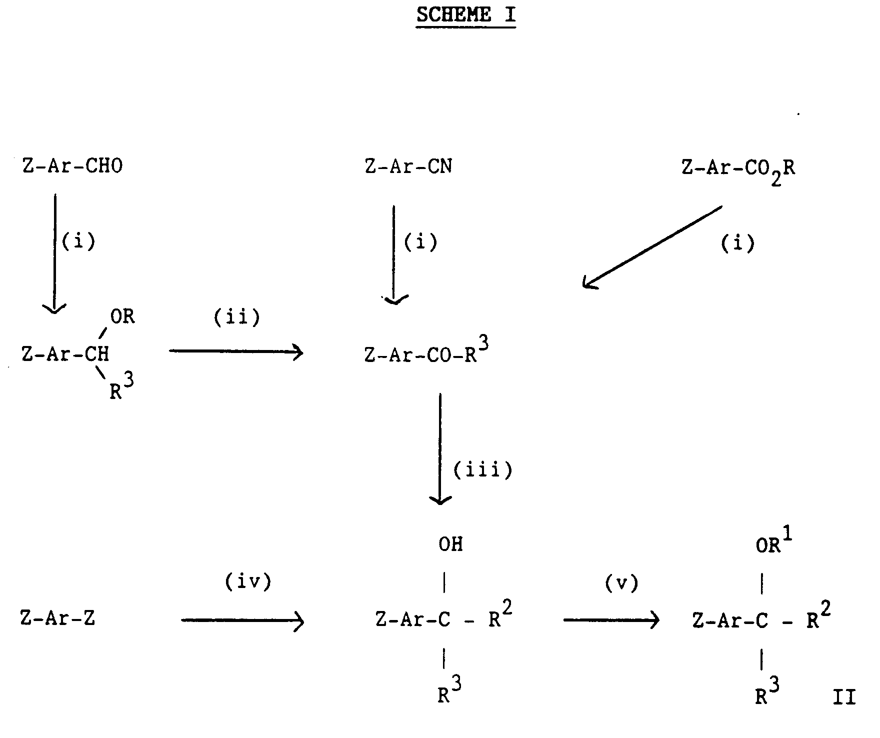

### Reagents

(i) R³Li or R³MgZ, THF
(ii) DDQ or MnO₂
(iii) R²Li or R²MgZ, THF;
(iv) BuLi or Mg, THF; R²COR³, THF
(v) R¹Z, base

Note R = (1-4C)alkyl such as Me or Et

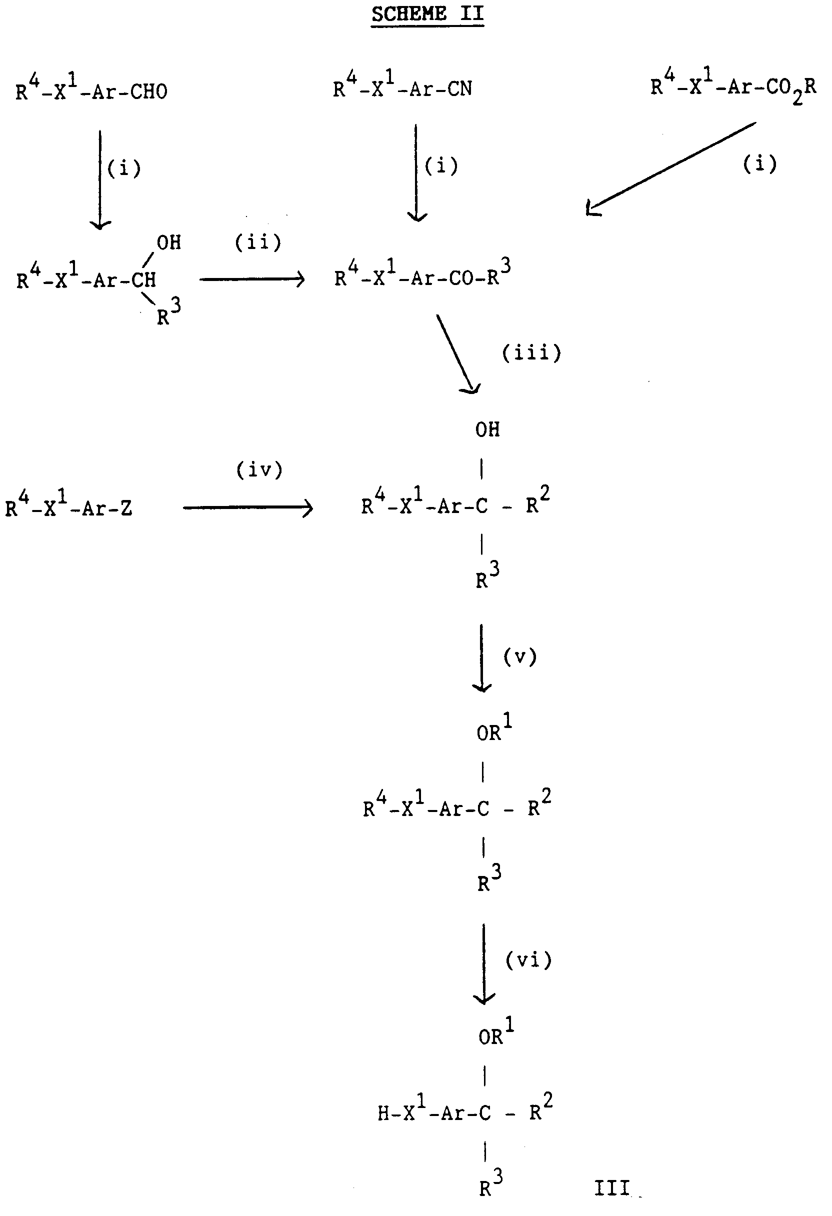

### Reagents

(i) to (v) as in Scheme I
(vi) Conventional removal of the protecting group R⁴ which is, e.g, COMe, THP, CH₂Ph or Me.

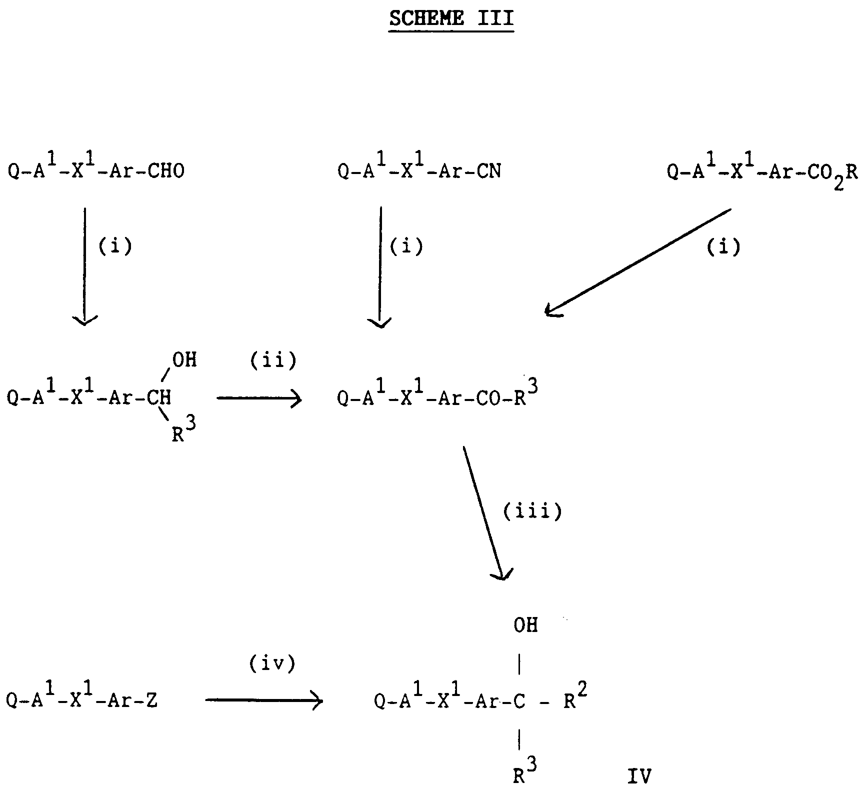

### Reagents

(i) to (iv) as in Scheme I

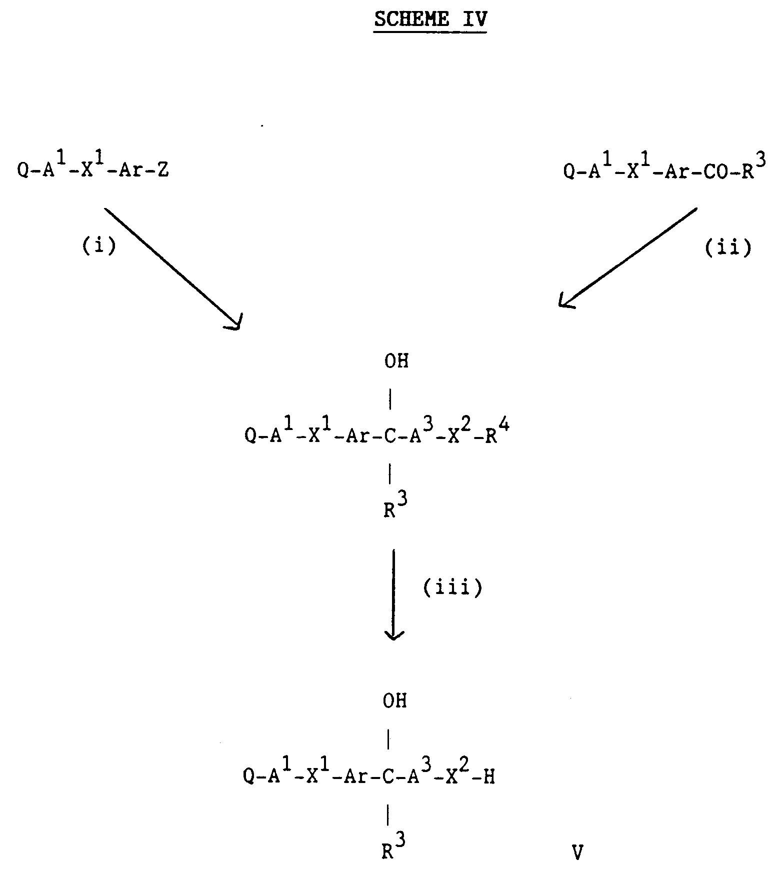

### Reagents

(i) BuLi or Mg, THF; R³-CO-A³-X²-R⁴
(ii) R⁴-X²-A³-Li or R⁴-X²-A³-MgZ, THF
(iii) Conventional removal of the protecting group R⁴ which is, e.g. COMe, THP, CH₂Ph or Me.

## Claims (Claims for the following Contracting State(s): AT, BE, CH, DE, DK, FR, GB, IT, LI, LU, NL, SE)

1. A bicyclic heterocyclic derivative of the formula I 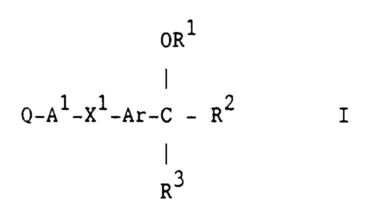 wherein Q is a 9-membered bicyclic heterocyclic moiety containing one or two nitrogen heteroatoms and optionally containing a further heteroatom selected from nitrogen, oxygen and sulphur, which heterocyclic moiety may optionally bear one or two oxo or thioxo substituents and up to four further substituents selected from halogeno, hydroxy, cyano, amino, (1-4C)alkyl, (1-4C)alkoxy, fluoro-(1-4C)alkyl, (1-4C)alkylamino, di-[(1-4C)alkyl]amino, amino-(1-4C)alkyl, (1-4C)alkylamino-(1-4C)alkyl, di-[(1-4C)alkyl]amino-(1-4C)alkyl, phenyl and phenyl-(1-4C)alkyl, and wherein said phenyl or phenyl-(1-4C)alkyl substituent may optionally bear a substituent selected from halogeno, (1-4C)alkyl and (1-4C)alkoxy;
wherein A¹ is a direct link to X¹ or is (1-3C)alkylene;
wherein X¹ is oxy, thio, sulphinyl, sulphonyl or imino;
wherein Ar is phenylene which may optionally bear one or two substituents selected from halogeno, hydroxy, amino, nitro, cyano, carbamoyl, ureido, (1-4C)alkyl, (1-4C)alkoxy, (1-4C)alkylamino, di-[(1-4C)alkyl]amino, fluoro-(1-4C)alkyl and (2-4C)alkanoylamino; or Ar is pyridylene;
wherein R¹ is (1-4C)alkyl, (3-4C)alkenyl or (3-4C)alkynyl; and
wherein R² and R³ together form a group of the formula -A²-X²-A³-which, together with the carbon atom to which A² and A³ are attached, defines a ring having 5 to 7 ring atoms, wherein A² and A³, which may be the same or different, each is (1-3C)alkylene and X² is oxy, thio, sulphinyl or sulphonyl, and which ring may bear one, two or three substituents, which may be the same or different, selected from hydroxy, (1-4C)alkyl and (1-4C)alkoxy;
or wherein R¹ and R² together form a group of the formula -A²-X²-A³-which, together with the oxygen atom to which A² is attached and with the carbon atom to which A³ is attached, defines a ring having 5 to 7 ring atoms, wherein A² and A³, which may be the same or different, each is (1-3C)alkylene and X² is oxy, thio, sulphinyl or sulphonyl, and which ring may bear one, two or three (1-4C)alkyl substituents, and wherein R³ is (1-4C)alkyl, (2-4C)alkenyl or (2-4C)alkynyl; or a pharmaceutically-acceptable salt thereof.

2. A bicyclic heterocyclic derivative of the formula I as claimed in claim 1
wherein Q is indolyl, indolinyl, benzimidazolyl,
2,3-dihydrobenzimidazolyl, 1H-indazolyl, 2,3-dihydro-1H-indazolyl, benzoxazolyl, 2,3-dihydrobenzoxazolyl, benzothiazolyl or 2,3-dihydrobenzothiazolyl, which may optionally bear one or two oxo or thioxo substituents and up to four substituents selected from fluoro, chloro, bromo, hydroxy, cyano, amino, methyl, ethyl, propyl, methoxy, trifluoromethyl, 2-fluoroethyl, 2,2,2-trifluoroethyl, 2-methylaminoethyl, 2-dimethylaminoethyl, phenyl and benzyl, and wherein said phenyl or benzyl substituent may optionally bear a substituent selected from chloro, methyl and methoxy;
A¹ is a direct link to X¹ or is methylene;
X¹ is oxy, thio, sulphinyl or sulphonyl;
Ar is 1,3-phenylene or 1,4-phenylene which may optionally bear one or two substituents selected from fluoro, chloro, hydroxy, amino, nitro, ureido, methoxy, dimethylamino, trifluoromethyl and acetamido; or Ar is 3,5-pyridylene;
R¹ is methyl, ethyl, allyl or 2-propynyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl, ethyl, propyl, methoxy and ethoxy;
or R¹ and R² together form a group of the formula -A²-X²-A³- which, together with the oxygen atom to which A² is attached and with the carbon atom to which A³ is attached, defines a ring having 5 or 6 ring atoms, wherein A² is methylene, A³ is methylene or ethylene, and X² is oxy, and which ring may bear one, two or three substituents selected from methyl, ethyl and propyl, and R³ is methyl or ethyl; or a pharmaceutically-acceptable salt thereof.

3. A bicyclic heterocyclic derivative of the formula I as claimed in claim 1
wherein Q is 2-oxoindolin-5-yl, 2,3-dioxoindolin-5-yl,
2-oxo-2,3-dihydrobenzimidazol-5-yl, 2-oxo-2,3-dihydrobenzoxazol-6-yl or 2-oxo-2,3-dihydrobenzothiazol-6-yl which may optionally bear one, two or three substituents selected from fluoro, chloro, hydroxy, methyl, ethyl, propyl, 2-fluoroethyl, 2-dimethylaminoethyl, phenyl and benzyl;
A¹ is a direct link to X¹, or is methylene;
X¹ is oxy, thio, sulphinyl or sulphonyl;
Ar is 1,3-phenylene or 1,4-phenylene which may optionally bear one or two substituents selected from fluoro, hydroxy, amino, nitro, ureido, methoxy, dimethylamino, trifluoromethyl and acetamido; or
Ar is 3,5-pyridylene;
R¹ is methyl, ethyl, allyl or 2-propynyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl and ethyl;
or R¹ and R² together form a group of the formula -A²-X²-A³- which, together with the oxygen atom to which A² is attached and with the carbon atom to which A³ is attached, defines a ring having 5 ring atoms, wherein A² is methylene, A³ is methylene, and X² is oxy, and which ring may bear one, two or three substituents selected from methyl and ethyl, and R³ is methyl or ethyl;
or a pharmaceutically-acceptable salt thereof.

4. A bicyclic heterocyclic derivative of the formula I as claimed in claim 1
wherein Q is 1,3,3-trimethyl-2-oxoindolin-5-yl, 1,3-dimethyl-2-oxo-2,3-dihydrobenzimidazol-5-yl or 3-methyl-2-oxo-2,3-dihydrobenzothiazol-6-yl;
A¹ is a direct link to X¹;
X¹ is thio;
Ar is 1,3-phenylene or 5-fluoro-1,3-phenylene;
R¹ is methyl; and
R² and R³ together form a group of the formula -A²-X²-A³- which, together with the carbon atom to which A² and A³ are attached, defines a ring having 6 ring atoms, wherein each of A² and A³ is ethylene and X² is oxy, and which ring may bear a methyl or ethyl substituent alpha to X²;
or a pharmaceutically-acceptable salt thereof.

5. A bicyclic heterocyclic derivative of the formula I as claimed in claim 1
wherein Q is 1,3,3-trimethyl-2-oxoindolin-5-yl, 1,3-dimethyl-2-oxo-2,3-dihydrobenzimidazol-5-yl, 3-methyl-2-oxo-2,3-dihydrobenzothiazol-6-yl or 3,3-difluoro-1-methyl-2-oxoindolin-5-yl;
A¹ is a direct link to X¹;
X¹ is thio;
Ar is 1,3-phenylene or 5-fluoro-1,3-phenylene;
R¹ is methyl; and
R² and R³ together form a group of the formula -A²-X²-A³- which,
together with the carbon atom to which A² and A³ are attached, defines a ring having 6 ring atoms, wherein each of A² and A³ is ethylene and X² is oxy, and which ring may bear a methyl or ethyl substituent alpha to X²;
or a pharmaceutically-acceptable salt thereof.

6. A bicyclic heterocyclic derivative of the formula I, or a pharmaceutically-acceptable salt thereof as defined in claim 1, selected from the group consisting of:-
4-methoxy-4-[3-(1,3,3-trimethyl-2-oxoindolin-5-ylthio)phenyl]tetrahydropyran,
4-[3-(1,3-dimethyl-2-oxo-2,3-dihydrobenzimidazol-5-ylthio)phenyl]-4-methoxytetrahydropyran and 4-methoxy-4-[3-(3-methyl-2-oxo-2,3-dihydrobenzothiazol-6-ylthio)phenyl]tetrahydropyran.

7. A bicyclic heterocyclic derivative of the formula I, or a pharmaceutically-acceptable salt thereof as defined in claim 1, selected from the group consisting of:-
4-[5-fluoro-3-(3-methyl-2-oxo-2,3-dihydrobenzothiazol-6-ylmethoxy)-phenyl]-4-methoxytetrahydropyran,
4-[5-fluoro-3-(1,3-dimethyl-2-oxo-2,3-dihydrobenzimidazol-5-ylmethoxy)phenyl]-4-methoxytetrahydropyran and
4-[5-fluoro-3-(3,3-difluoro-1-methyl-2-oxoindolin-5-ylmethoxy)phenyl]-4-methoxytetrahydropyran.

8. A process for the preparation of a bicyclic heterocyclic derivative of the formula I, or a pharmaceutically-acceptable salt thereof, as claimed in claim 1 which comprises:-
(a) the coupling of a compound of the formula Q-A¹-X¹-H with a compound of the formula II 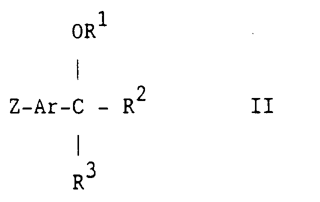 wherein Z is a displaceable group; provided that, when there is an amino, alkylamino or hydroxy group in Q, Ar, R² or R³, any amino, alkylamino or hydroxy group may be protected by a conventional protecting group or alternatively any such group need not be protected; whereafter any undesired protecting group in Q, Ar, R² or R³ is removed by conventional means;
(b) the coupling of a compound of the formula III 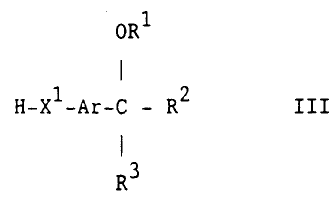 with a compound of the formula Q-A¹-Z wherein Z is a displaceable group; provided that, when there is an amino, alkylamino or hydroxy group in Q, Ar, R² or R³, any amino, alkylamino or hydroxy group may be protected by a conventional protecting group or alternatively any such group need not be protected, whereafter any undesired protecting group in Q, Ar, R² or R³ is removed by conventional means;
(c) the alkylation of a compound of the formula IV 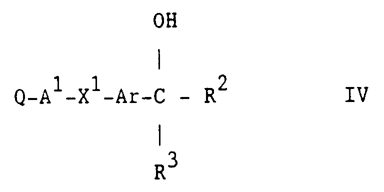 with a compound of the formula R¹-Z, wherein R¹ and Z have the meanings defined hereinbefore; provided that, when there is an amino, imino, alkylamino or hydroxy group in Q, X¹ , Ar, R² or R³ any amino, imino, alkylamino or hydroxy group may be protected by a conventional protecting group or alternatively any such group need not be protected, whereafter any undesired protecting group in Q, X¹, Ar, R² or R³ is removed by conventional means;
(d) for the production of those compounds of the formula I wherein R¹ and R² together form a group of the formula -A²-X²-A³-which, together with the oxygen atom to which A² is attached, defines a ring having 5 to 7 ring atoms and wherein A², X² and A³ have the meanings defined hereinbefore, and wherein R³ has the meaning defined hereinbefore; the cyclisation of a compound of the formula V 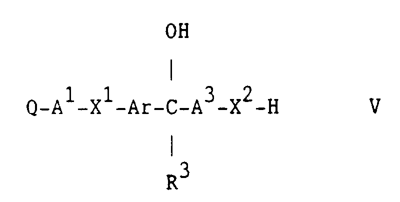 upon reaction with an appropriate aldehyde or ketone, or with a hemiacetal or acetal thereof, or with a compound of the formula Z-A²-Z, wherein Z has the meaning defined hereinbefore; provided that, when there is an amino, imino, alkylamino or hydroxy group in Q, X¹ or Ar, any amino, imino, alkylamino or hydroxy group is protected by a conventional protecting group, whereafter any undesired protecting group in Q, X¹ or Ar is removed by conventional means;
(e) for the production of those compounds of the formula I wherein X¹ is a sulphinyl or sulphonyl group, wherein R² and R³ together form a group of the formula -A²-X²-A³- and X² is a sulphinyl or sulphonyl group or wherein R¹ and R² together form a group of the formula -A²-X²-A³- and X² is a sulphinyl or sulphonyl group, the oxidation of a compound of the formula I wherein X¹ is a thio group, wherein R² and R³ together form a group of the formula -A²-X²-A³- and X² is a thio group or wherein R¹ and R² together form a group of the formula -A²-X²-A³- and X² is a thio group;
(f) for the production of those compounds of the formula I wherein Ar bears an alkanoylamino substituent, the acylation of a compound of the formula I wherein Ar bears an amino substituent; or
(g) for the production of those compounds of the formula I wherein Q bears an alkyl or substituted alkyl substituent on an available nitrogen atom, or wherein Ar bears an alkoxy substituent, the alkylation of a compound of the formula I wherein Q bears a hydrogen atom on said available nitrogen atom, or wherein Ar bears a hydroxy substituent; and
when a pharmaceutically-acceptable salt of a novel compound of the formula I is required, it may be obtained by reaction of said compound with a suitable acid or base using a conventional procedure.

9. A pharmaceutical composition which comprises a bicyclic heterocyclic derivative of the formula I, or a pharmaceutically-acceptable salt thereof, as claimed in any one of claims 1 to 7 in association with a pharmaceutically-acceptable diluent or carrier.

10. The use of a bicyclic heterocyclic derivative of the formula I, or a pharmaceutically-acceptable salt thereof, as claimed in any one of claims 1 to 7 in the production of a new medicament for use in a leukotriene mediated disease or medical condition.

## Claims (Claims for the following Contracting State(s): GR, ES)

1. A process for the preparation of a bicyclic heterocyclic derivative of the formula I 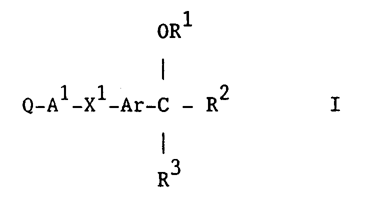 wherein Q is a 9-membered bicyclic heterocyclic moiety containing one or two nitrogen heteroatoms and optionally containing a further heteroatom selected from nitrogen, oxygen and sulphur, which heterocyclic moiety may optionally bear one or two oxo or thioxo substituents and up to four further substituents selected from halogeno, hydroxy, cyano, amino, (1-4C)alkyl, (1-4C)alkoxy, fluoro-(1-4C)alkyl, (1-4C)alkylamino, di-[(1-4C)alkyl]amino, amino-(1-4C)alkyl, (1-4C)alkylamino-(1-4C)alkyl, di-[(1-4C)alkyl]amino-(1-4C)alkyl, phenyl and phenyl-(1-4C)alkyl, and wherein said phenyl or phenyl-(1-4C)alkyl substituent may optionally bear a substituent selected from halogeno, (1-4C)alkyl and (1-4C)alkoxy;
wherein A¹ is a direct link to X¹ or is (1-3C)alkylene;
wherein X¹ is oxy, thio, sulphinyl, sulphonyl or imino;
wherein Ar is phenylene which may optionally bear one or two substituents selected from halogeno, hydroxy, amino, nitro, cyano, carbamoyl, ureido, (1-4C)alkyl, (1-4C)alkoxy, (1-4C)alkylamino, di-[(1-4C)alkyl]amino, fluoro-(1-4C)alkyl and (2-4C)alkanoylamino; or Ar is pyridylene;
wherein R¹ is (1-4C)alkyl, (3-4C)alkenyl or (3-4C)alkynyl; and
wherein R² and R³ together form a group of the formula -A²-X²-A³-which, together with the carbon atom to which A² and A³ are attached, defines a ring having 5 to 7 ring atoms, wherein A² and A³, which may be the same or different, each is (1-3C)alkylene and X² is oxy, thio, sulphinyl or sulphonyl, and which ring may bear one, two or three substituents, which may be the same or different, selected from hydroxy, (1-4C)alkyl and (1-4C)alkoxy;
or wherein R¹ and R² together form a group of the formula -A²-X²-A³-which, together with the oxygen atom to which A² is attached and with the carbon atom to which A³ is attached, defines a ring having 5 to 7 ring atoms, wherein A² and A³, which may be the same or different, each is (1-3C)alkylene and X² is oxy, thio, sulphinyl or sulphonyl, and which ring may bear one, two or three (1-4C)alkyl substituents, and wherein R³ is (1-4C)alkyl, (2-4C)alkenyl or (2-4C)alkynyl; or a pharmaceutically-acceptable salt thereof;
characterised by
(a) the coupling of a compound of the formula Q-A¹-X¹-H with a compound of the formula II 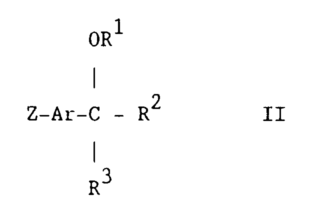 wherein Z is a displaceable group; provided that, when there is an amino, alkylamino or hydroxy group in Q, Ar, R² or R³, any amino, alkylamino or hydroxy group may be protected by a conventional protecting group or alternatively any such group need not be protected; whereafter any undesired protecting group in Q, Ar, R² or R³ is removed by conventional means;
(b) the coupling of a compound of the formula III 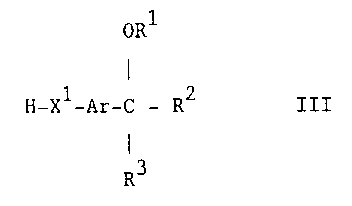 with a compound of the formula Q-A¹-Z wherein Z is a displaceable group; provided that, when there is an amino, alkylamino or hydroxy group in Q, Ar, R² or R³, any amino, alkylamino or hydroxy group may be protected by a conventional protecting group or alternatively any such group need not be protected, whereafter any undesired protecting group in Q, Ar, R² or R³ is removed by conventional means;
(c) the alkylation of a compound of the formula IV 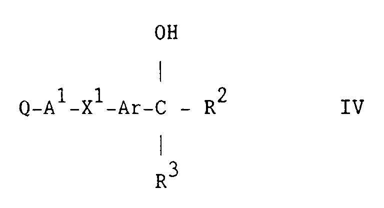 with a compound of the formula R¹-Z, wherein R¹ and Z have the meanings defined hereinbefore; provided that, when there is an amino, imino, alkylamino or hydroxy group in Q, X¹, Ar, R² or R³ any amino, imino, alkylamino or hydroxy group may be protected by a conventional protecting group or alternatively any such group need not be protected, whereafter any undesired protecting group in Q, X¹, Ar, R² or R³ is removed by conventional means;
(d) for the production of those compounds of the formula I wherein R¹ and R² together form a group of the formula -A²-X²-A³-which, together with the oxygen atom to which A² is attached, defines a ring having 5 to 7 ring atoms and wherein A², X² and A³ have the meanings defined hereinbefore, and wherein R³ has the meaning defined hereinbefore; the cyclisation of a compound of the formula V 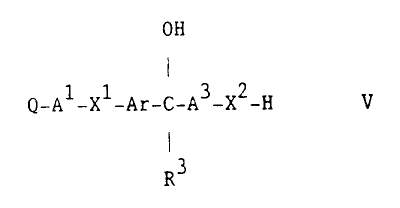 upon reaction with an appropriate aldehyde or ketone, or with a hemiacetal or acetal thereof, or with a compound of the formula Z-A²-Z, wherein Z has the meaning defined hereinbefore; provided that, when there is an amino, imino, alkylamino or hydroxy group in Q, X¹ or Ar, any amino, imino, alkylamino or hydroxy group is protected by a conventional protecting group, whereafter any undesired protecting group in Q, X¹ or Ar is removed by conventional means;
(e) for the production of those compounds of the formula I wherein X¹ is a sulphinyl or sulphonyl group, wherein R² and R³ together form a group of the formula -A²-X²-A³- and X² is a sulphinyl or sulphonyl group or wherein R¹ and R² together form a group of the formula -A²-X²-A³- and X² is a sulphinyl or sulphonyl group, the oxidation of a compound of the formula I wherein X¹ is a thio group, wherein R² and R³ together form a group of the formula -A²-X²-A³- and X² is a thio group or wherein R¹ and R² together form a group of the formula -A²-X²-A³- and X² is a thio group;
(f) for the production of those compounds of the formula I wherein Ar bears an alkanoylamino srnbstituent, the acylation of a compound of the formula I wherein Ar bears an amino substituent; or
(g) for the production of those compounds of the formula I wherein Q bears an alkyl or substituted alkyl substituent on an available nitrogen atom, or wherein Ar bears an alkoxy substituent, the alkylation of a compound of the formula I wherein Q bears a hydrogen atom on said available nitrogen atom, or wherein Ar bears a hydroxy substituent; and
when a pharmaceutically-acceptable salt of a novel compound of the formula I is required, it may be obtained by reaction of said compound with a suitable acid or base using a conventional procedure.

2. A process as claimed in claim 1 for the preparation of a bicyclic heterocyclic derivative of the formula I wherein Q is indolyl, indolinyl, benzimidazolyl, 2,3-dihydrobenzimidazolyl, 1H-indazolyl, 2,3-dihydro-1H-indazolyl, benzoxazolyl, 2,3-dihydrobenzoxazolyl, benzothiazolyl or 2,3-dihydrobenzothiazolyl, which may optionally bear one or two oxo or thioxo substituents and up to four substituents selected from fluoro, chloro, bromo, hydroxy, cyano, amino, methyl, ethyl, propyl, methoxy, trifluoromethyl, 2-fluoroethyl, 2,2,2-trifluoroethyl, 2-methylaminoethyl, 2-dimethylaminoethyl, phenyl and benzyl, and wherein said phenyl or benzyl substituent may optionally bear a substituent selected from chloro, methyl and methoxy;
A¹ is a direct link to X¹ or is methylene;
X¹ is oxy, thio, sulphinyl or sulphonyl;
Ar is 1,3-phenylene or 1,4-phenylene which may optionally bear one or two substituents selected from fluoro, chloro, hydroxy, amino, nitro, ureido, methoxy, dimethylamino, trifluoromethyl and acetamido; or Ar is 3,5-pyridylene;
R¹ is methyl, ethyl, allyl or 2-propynyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl, ethyl, propyl, methoxy and ethoxy;
or R¹ and R² together form a group of the formula -A²-X²-A³- which, together with the oxygen atom to which A² is attached and with the carbon atom to which A³ is attached, defines a ring having 5 or 6 ring atoms, wherein A² is methylene, A³ is methylene or ethylene, and X² is oxy, and which ring may bear one, two or three substituents selected from methyl, ethyl and propyl, and R³ is methyl or ethyl; or a pharmaceutically-acceptable salt thereof.

3. A process as claimed in claim 1 for the preparation of a bicyclic heterocyclic derivative of the formula I wherein Q is 2-oxoindolin-5-yl, 2,3-dioxoindolin-5-yl, 2-oxo-2,3-dihydrobenzimidazol-5-yl, 2-oxo-2,3-dihydrobenzoxazol-6-yl or 2-oxo-2,3-dihydrobenzothiazol-6-yl which may optionally bear one, two or three substituents selected from fluoro, chloro, hydroxy, methyl, ethyl, propyl, 2-fluoroethyl, 2-dimethylaminoethyl, phenyl and benzyl; A¹ is a direct link to X¹, or is methylene;
X¹ is oxy, thio, sulphinyl or sulphonyl;
Ar is 1,3-phenylene or 1,4-phenylene which may optionally bear one or two substituents selected from fluoro, hydroxy, amino, nitro, ureido, methoxy, dimethylamino, trifluoromethyl and acetamido; or
Ar is 3,5-pyridylene;
R¹ is methyl, ethyl, allyl or 2-propynyl; and
R² and R³ together form a group of the formula -A²-X²-A³- which, together with the carbon atom to which A² and A³ are attached, defines a ring having 5 or 6 ring atoms, wherein A² is ethylene, A³ is methylene or ethylene, and X² is oxy, and which ring may bear one or two substituents selected from methyl and ethyl;
or R¹ and R² together form a group of the formula -A²-X²-A³- which, together with the oxygen atom to which A² is attached and with the carbon atom to which A³ is attached, defines a ring having 5 ring atoms, wherein A² is methylene, A³ is methylene, and X² is oxy, and which ring may bear one, two or three substituents selected from methyl and ethyl, and R³ is methyl or ethyl;
or a pharmaceutically-acceptable salt thereof.

4. A process as claimed in claim 1 for the preparation of a bicyclic heterocyclic derivative of the formula I wherein Q is 1,3,3-trimethyl-2-oxoindolin-5-yl, 1,3-dimethyl-2-oxo-2,3-dihydrobenzimidazol-5-yl or 3-methyl-2-oxo-2,3-dihydrobenzothiazol-6-yl;
A¹ is a direct link to X¹;
X¹ is thio;
Ar is 1,3-phenylene or 5-fluoro-1,3-phenylene;
R¹ is methyl; and
R² and R³ together form a group of the formula -A²-X²-A³- which, together with the carbon atom to which A² and A³ are attached, defines a ring having 6 ring atoms, wherein each of A² and A³ is ethylene and X² is oxy, and which ring may bear a methyl or ethyl substituent alpha to X²;
or a pharmaceutically-acceptable salt thereof.

5. A process as claimed in claim 1 for the preparation of a bicyclic heterocyclic derivative of the formula I wherein Q is 1,3,3-trimethyl-2-oxoindolin-5-yl, 1,3-dimethyl-2-oxo-2,3-dihydrobenzimidazol-5-yl, 3-methyl-2-oxo-2,3-dihydrobenzothiazol-6-yl or 3,3-difluoro-1-methyl-2-oxoindolin-5-yl;
A¹ is a direct link to X¹;
X¹ is thio;
Ar is 1,3-phenylene or 5-fluoro-1,3-phenylene;
R¹ is methyl; and
R² and R³ together form a group of the formula -A²-X²-A³- which, together with the carbon atom to which A² and A³ are attached, defines a ring having 6 ring atoms, wherein each of A² and A³ is ethylene and X² is oxy, and which ring may bear a methyl or ethyl substituent alpha to X²;
or a pharmaceutically-acceptable salt thereof.

6. A process for the preparation of a pharmaceutical composition characterised by bringing into association a bicyclic heterocyclic derivative of the formula I, or a pharmaceutically-acceptable salt thereof, as defined in any one of claims 1 to 5 and a pharmaceutically-acceptable diluent or carrier.

## Patentansprüche (Patentansprüche für folgende(n) Vertragsstaat(en): AT, BE, CH, DE, DK, FR, GB, IT, LI, LU, NL, SE)

1. Bicyclisches heterocyclisches Derivat mit der Formel I: 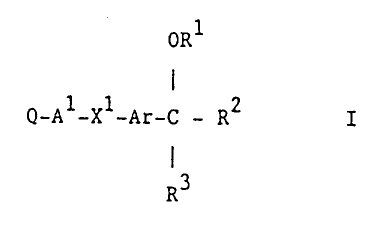 in der
Q für einen 9-gliedrigen bicyclischen heterocyclischen Rest steht, der ein oder zwei Stickstoff-Heteroatome und gegebenenfalls ein weiteres unter Stickstoff, Sauerstoff und Schwefel ausgewähltes Heteroatom enthält, wobei der heterocyclische Rest gegebenenfalls einen oder zwei Oxo- oder Thioxo-Substituenten und bis zu vier weitere Substituenten tragen kann, die unter Halogen, Hydroxy, Cyano, Amino, (1-4C)Alkyl, (1-4C)Alkoxy, Fluor-(1-4C)alkyl, (1-4C)Alkylamino, Di-[(1-4C)alkyl]amino, Amino-(1-4C)alkyl, (1-4C)Alkylamino-(1-4C)alkyl, Di-[(1-4C)alkyl]amino-(1-4C)alkyl, Phenyl und Phenyl-(1-4C)alkyl ausgewählt sind, wobei der Phenyl- oder der Phenyl-(1-4C)alkyl-Substituent gegebenenfalls einen Substituenten tragen kann, der unter Halogen, (1-4C)Alkyl und (1-4C)Alkoxy ausgewählt ist;
A¹ für eine Direktbindung zu X¹ oder für (1-3C)Alkylen steht;
X¹ für Oxy, Thio, Sulfinyl, Sulfonyl oder Imino steht; Ar für Phenylen steht, das gegebenenfalls einen oder zwei Substituenten tragen kann, die unter Halogen, Hydroxy, Amino, Nitro, Cyano, Carbamoyl, Ureido, (1-4C)Alkyl, (1-4C)Alkoxy, (1-4C)Alkylamino, Di-[(1-4C)alkyl]amino, Fluor-(1-4C)alkyl und (2-4C)Alkanoylamino ausgewählt sind; oder
Ar für Pyridylen steht;
R¹ für (1-4C)Alkyl, (3-4C)Alkenyl oder (3-4C)Alkinyl steht; und
R² und R³ zusammen eine Gruppe mit der Formel -A²-X²-A³- bilden, die zusammen mit dem Kohlenstoffatom, an das A² und A³ gebunden sind, einen Ring mit 5 bis 7 Ringatomen definiert, wobei A² und A³, die gleich oder unterschiedlich sein können, jeweils für (1-3C)Alkylen stehen und X² für Oxy, Thio, Sulfinyl oder Sulfonyl steht, und wobei der Ring einen, zwei oder drei Substituenten tragen kann, die gleich oder unterschiedlich sein können, die unter Hydroxy, (1-4C)Alkyl und (1-4C)Alkoxy ausgewählt sind; oder in der
R¹ und R² zusammen eine Gruppe mit der Formel -A²-X²-A³- bilden, die zusammen mit dem Sauerstoffatom, an das A² gebunden ist, und mit dem Kohlenstoffatom, an das A³ gebunden ist, einen Ring mit 5 bis 7 Ringatomen definiert, wobei A² und A³, die gleich oder unterschiedlich sein können, jeweils für (1-3C)Alkylen stehen und X² für Oxy, Thio, Sulfinyl oder Sulfonyl steht, und wobei der Ring einen, zwei oder drei (1-4C)Alkyl-Substituenten tragen kann und R³ für (1-4C)Alkyl, (2-4C)Alkenyl oder (2-4C)Alkinyl steht;
oder ein pharmazeutisch geeignetes Salz davon.

2. Bicyclisches heterocyclisches Derivat mit der Formel I nach Anspruch 1, in der
Q Indolyl, Indolinyl, Benzimidazolyl, 2,3-Dihydrobenzimidazolyl, 1H-Indazolyl, 2,3-Dihydro-1H-indazolyl, Benzoxazolyl, 2,3-Dihydrobenzoxazolyl, Benzothiazolyl oder 2,3-Dihydrobenzothiazolyl steht, das gegebenenfalls einen oder zwei Oxo- oder Thioxo-Substituenten und bis zu vier Substituenten tragen kann, die unter Fluor, Chlor, Brom, Hydroxy, Cyano, Amino, Methyl, Ethyl, Propyl, Methoxy, Trifluormethyl, 2-Fluorethyl, 2,2,2-Trifluorethyl, 2-Methylaminoethyl, 2-Dimethylaminoethyl, Phenyl und Benzyl ausgewählt sind, wobei der Phenyl- oder Benzyl-Substituent gegebenenfalls einen Substituenten tragen kann, der unter Chlor, Methyl und Methoxy ausgewählt ist;
A¹ für eine Direktbindung zu X¹ oder für Methylen steht;
X¹ für Oxy, Thio, Sulfinyl oder Sulfonyl steht;
Ar für 1,3-Phenylen oder 1,4-Phenylen steht, das gegebenenfalls einen oder zwei Substituenten tragen kann, die unter Fluor, Chlor, Hydroxy, Amino, Nitro, Ureido, Methoxy, Dimethylamino, Trifluormethyl und Acetamido ausgewählt sind; oder in der
Ar für 3,5-Pyridylen steht;
R¹ für Methyl, Ethyl, Allyl oder 2-Propinyl steht; und
R² und R³ zusammen eine Gruppe mit der Formel -A²-X²-A³- bilden, die zusammen mit dem Kohlenstoffatom, an das A² und A³ gebunden sind, einen Ring mit 5 oder 6 Ringatomen definiert, wobei A² für Ethylen steht, A³ für Methylen oder Ethylen steht und X² für Oxy steht, und wobei der Ring einen oder zwei Substituenten tragen kann, die unter Methyl, Ethyl, Propyl, Methoxy und Ethoxy ausgewählt sind; oder in der
R¹ und R² zusammen eine Gruppe mit der Formel -A²-X²-A³- bilden, die zusammen mit dem Sauerstoffatom, an das A² gebunden ist, und mit dem Kohlenstoffatom, an das A³ gebunden ist, einen Ring mit 5 oder 6 Ringatomen definiert, wobei A² für Methylen steht, A³ für Methylen oder Ethylen steht und X² für Oxy steht, und wobei der Ring einen, zwei oder drei Substituenten tragen kann, die unter Methyl, Ethyl und Propyl ausgewählt sind, und R³ für Methyl oder Ethyl steht;
oder ein pharmazeutisch geeignetes Salz davon.

3. Bicyclisches heterocyclisches Derivat mit der Formel I nach Anspruch 1, in der
Q für 2-Oxoindolin-5-yl, 2,3-Dioxoindolin-5-yl, 2-Oxo-2,3-dihydrobenzimidazol-5-yl, 2-Oxo-2,3-dihydrobenzoxazol-6-yl oder 2-Oxo-2,3-dihydrobenzothiazol-6-yl steht, das gegebenenfalls einen, zwei oder drei Substituenten tragen kann, die unter Fluor, Chlor, Hydroxy, Methyl, Ethyl, Propyl, 2-Fluorethyl, 2-Dimethylaminoethyl, Phenyl und Benzyl ausgewählt sind; A¹ für eine Direktbindung zu X¹ oder für Methylen steht;
X¹ für Oxy, Thio, Sulfinyl oder Sulfonyl steht; Ar für 1,3-Phenylen oder 1,4-Phenylen steht, das gegebenenfalls einen oder zwei Substituenten tragen kann, die unter Fluor, Hydroxy, Amino, Nitro, Ureido, Methoxy, Dimethylamino, Trifluormethyl und Acetamido ausgewählt sind; oder in der
Ar für 3,5-Pyridylen steht;
R¹ für Methyl, Ethyl, Allyl oder 2-Propinyl steht; und
R² und R³ zusammen eine Gruppe mit der Formel -A²-X²-A³- bilden, die zusammen mit dem Kohlenstoffatom, an das A² und A³ gebunden sind, einen Ring mit 5 oder 6 Ringatomen definiert, wobei A² für Ethylen steht, A³ für Methylen oder Ethylen steht und X² für Oxy steht, und wobei der Ring einen oder zwei Substituenten tragen kann, die unter Methyl und Ethyl ausgewählt sind; oder in der
R¹ und R² zusammen eine Gruppe mit der Formel -A²-X²-A³- bilden, die zusammen mit dem Sauerstoffatom, an das A² gebunden ist, und mit dem Kohlenstoffatom, an das A³ gebunden ist, einen Ring mit 5 Ringatomen definiert, wobei A² für Methylen steht, A³ für Methylen steht und X² für Oxy steht, und wobei der Ring einen, zwei oder drei Substituenten tragen kann, die unter Methyl und Ethyl ausgewählt sind, und R³ für Methyl oder Ethyl steht;
oder ein pharmazeutisch geeignetes Salz davon.

4. Bicyclisches heterocyclisches Derivat mit der Formel I nach Anspruch 1, in der
Q für 1,3,3-Trimethyl-2-oxoindolin-5-yl, 1,3-Dimethyl-2-oxo-2,3-dihydrobenzimidazol-5-yl oder 3-Methyl-2-oxo-2,3-dihydrobenzothiazol-6-yl steht;
A¹ für eine Direktbindung zu X¹ steht;
X¹ für Thio steht;
Ar für 1,3-Phenylen oder 5-Fluor-1,3-phenylen steht;
R¹ für Methyl steht;
R² und R³ zusammen eine Gruppe mit der Formel -A²-X²-A³- bilden, die zusammen mit dem Kohlenstoffatom, an das A² und A³ gebunden sind, einen Ring mit 6 Ringatomen definiert, wobei A² und A³ jeweils für Ethylen stehen und X² für Oxy steht, und wobei der Ring einen Methyl- oder Ethyl-Substituenten alpha zu X² tragen kann;
oder ein pharmazeutisch geeignetes Salz davon.

5. Bicyclisches heterocyclisches Derivat mit der Formel I nach Anspruch 1, in der
Q für 1,3,3-Trimethyl-2-oxoindolin-5-yl, 1,3-Dimethyl-2-oxo-2,3-dihydrobenzimidazol-5-yl, 3-Methyl-2-oxo-2,3-dihydrobenzothiazol-6-yl oder 3,3-Difluor-1-methyl-2-oxoindolin-5-yl steht;
A¹ für eine Direktbindung zu X¹ steht;
X¹ für Thio steht;
Ar für 1,3-Phenylen oder 5-Fluor-1,3-phenylen steht;
R¹ für Methyl steht;
R² und R³ zusammen eine Gruppe mit der Formel -A²-X²-A³- bilden, die zusammen mit dem Kohlenstoffatom, an das A² und A³ gebunden sind, einen Ring mit 6 Ringatomen definiert, wobei A² und A³ jeweils für Ethylen stehen und X² für Oxy steht, und wobei der Ring einen Methyl- oder Ethyl-Substituenten alpha zu X² tragen kann;
oder ein pharmazeutisch geeignetes Salz davon.

6. Bicyclisches heterocyclisches Derivat mit der Formel I, oder ein pharmazeutisch geeignetes Salz davon, nach Anspruch 1, das aus der folgenden Gruppe ausgewählt ist:-
4-Methoxy-4-[3-(1,3,3-trimethyl-2-oxoindolin-5-ylthio)phenyl]tetrahydropyran,
4-[3-(1,3-Dimethyl-2-oxo-2,3-dihydrobenzimidazol-5-ylthio)phenyl]-4-methoxytetrahydropyran und
4-Methoxy-4-[3-(3-methyl-2-oxo-2,3-dihydrobenzothiazol-6-ylthio)phenyl]tetrahydropyran.

7. Bicyclisches heterocyclisches Derivat mit der Formel I, oder ein pharmazeutisch geeignetes Salz davon, nach Anspruch 1, das aus der folgenden Gruppe ausgewählt ist:-
4-[5-Fluor-3-(3-methyl-2-oxo-2,3-dihydrobenzothiazol-6-ylmethoxy)phenyl]-4-methoxytetrahydropyran,
4-[5-Fluor-3-(1,3-dimethyl-2-oxo-2,3-dihydrobenzimidazol-5-ylmethoxy)phenyl]-4-methoxytetrahydropyran und
4-[5-Fluor-3-(3,3-difluor-1-methyl-2-oxoindolin-5-ylmethoxy)phenyl]-4-methoxytetrahydropyran.

8. Verfahren zur Herstellung eines bicyclischen heterocyclischen Derivats mit der Formel I, oder eines pharmazeutisch geeigneten Salzes davon, nach Anspruch 1, bei dem:-
(a) eine Verbindung mit der Formel Q-A¹-X¹-H mit einer Verbindung mit der Formel II, 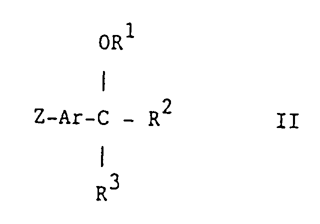 in der Z für eine austauschbare Gruppe steht, gekuppelt wird, und zwar mit der Maßgabe, daß jede Amino-, Alkylamino- oder Hydroxy-Gruppe mit einer üblichen Schutzgruppe geschützt sein kann, wenn eine Amino-, Alkylamino- oder Hydroxy-Gruppe in Q, Ar, R² oder R³ vorhanden ist, oder alternativ keine derartige Gruppe geschützt werden muß, woraufhin jede unerwünschte Schutzgruppe in Q, Ar, R² oder R³ mit üblichen Mitteln entfernt wird;
(b) eine Verbindung mit der Formel III 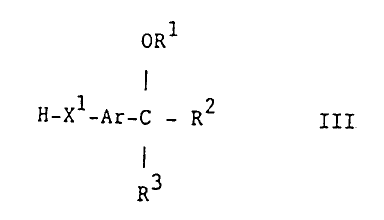 mit einer Verbindung mit der Formel Q-A¹-Z, in der Z für eine austauschbare Gruppe steht, gekuppelt wird, und zwar unter der Voraussetzung, daß jede Amino-, Alkylamino- oder Hydroxy-Gruppe mit einer üblichen Schutzgruppe geschützt sein kann, wenn eine Amino-, Alkylamino- oder Hydroxy-Gruppe in Q, Ar, R² oder R³ vorhanden ist, oder alternativ keine derartige Gruppe geschützt werden muß, woraufhin jede unerwünschte Schutzgruppe in Q, Ar, R² oder R³ mit üblichen Mitteln entfernt wird;
(c) eine Verbindung mit der Formel IV 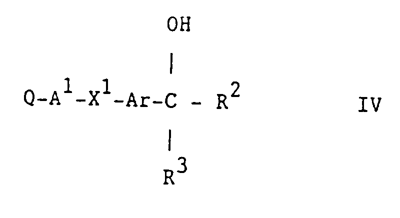 mit einer Verbindung mit der Formel R¹-Z, in der R¹ und Z die oben definierten Bedeutungen haben, alkyliert wird, und zwar unter der Voraussetzung, daß jede Amino-, Imino-, Alkylamino- oder Hydroxy-Gruppe mit einer üblichen Schutzgruppe geschützt sein kann, wenn eine Amino-, Imino-, Alkylamino- oder Hydroxy-Gruppe in Q, X¹, Ar, R² oder R³ vorhanden ist, oder alternativ keine derartige Gruppe geschützt werden muß, woraufhin jede unerwünschte Schutzgruppe in Q, X¹, Ar, R² oder R³ mit üblichen Mitteln entfernt wird;
(d) zur Herstellung derjenigen Verbindungen mit der Formel I, bei denen R¹ und R² zusammen eine Gruppe mit der Formel -A²-X²-A³- bilden, die zusammen mit dem Sauerstoffatom, an das A² gebunden ist, einen Ring mit 5 bis 7 Ringatomen definiert, wobei A², X² und A³ die oben definierten Bedeutungen haben, und wobei R³ die oben definierte Bedeutung hat, eine Verbindung mit der Formel V 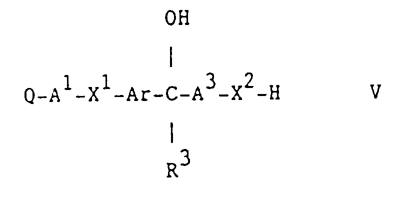 durch Umsetzung mit einem geeigneten Aldehyd oder Keton, oder mit einem Hemiacetal oder Acetal davon, oder mit einer Verbindung mit der Formel Z-A²-Z, in der Z die oben definierte Bedeutung hat, cyclisiert wird, und zwar unter der Voraussetzung, daß jede Amino-, Imino-, Alkylamino- oder Hydroxy-Gruppe mit einer üblichen Schutzgruppe geschützt ist, wenn eine Amino-, Imino-, Alkylamino- oder Hydroxy-Gruppe in Q, X¹ oder Ar vorhanden ist, woraufhin jede unerwünschte Schutzgruppe in Q, X¹ oder Ar mit üblichen Mitteln entfernt wird;
(e) zur Herstellung derjenigen Verbindungen mit der Formel I, bei denen X¹ für eine Sulfinyl- oder Sulfonyl-Gruppe steht, bei denen R² und R³ zusammen eine Gruppe mit der Formel -A²-X²-A³- bilden und X² für eine Sulfinyl- oder Sulfonyl-Gruppe steht, oder bei denen R¹ und R² zusammen eine Gruppe mit der Formel -A²-X²-A³- bilden und X² für eine Sulfinyl- oder Sulfonyl-Gruppe steht, eine Verbindung mit der Formel I, bei der X¹ für eine Thio-Gruppe steht, bei der R² und R³ zusammen eine Gruppe mit der Formel -A²-X²-A³- bilden und X² für eine Thio-Gruppe steht, oder bei der R¹ und R² zusammen eine Gruppe mit der Formel -A²-X²-A³- bilden und X² für eine Thio-Gruppe steht, oxidiert wird;
(f) zur Herstellung derjenigen Verbindungen mit der Formel I, bei denen Ar einen Alkanoylamino-Substituenten trägt, eine Verbindung mit der Formel I, in der Ar einen Amino-Substituenten trägt, acyliert wird;
(g) zur Herstellung derjenigen Verbindungen mit der Formel I, bei denen Q einen Alkyl- oder substituierten Alkyl-Substituenten an einem verfügbaren Stickstoffatom trägt, oder bei denen Ar einen Alkoxy-Substituenten trägt, eine Verbindung mit Formel I, bei der Q ein Wasserstoffatom an dem verfügbaren Stickstoffatom trägt, oder bei der Ar einen Hydroxy-Substituenten trägt, alkyliert wird; und
wenn ein pharmazeutisch geeignetes Salz einer neuen Verbindung mit der Formel I benötigt wird, dieses durch Umsetzung der Verbindung mit einer geeigneten Säure oder Base unter Anwendung eines üblichen Verfahrens erhältlich ist.

9. Pharmazeutische Zusammensetzung, die ein bicyclisches heterocyclisches Derivat mit der Formel I, oder ein pharmazeutisch geeignetes Salz davon, nach einem der Ansprüche 1 bis 7, in Verbindung mit einem pharmazeutisch geeigneten Streckmittel oder Trägermittel enthält.

10. Verwendung eines bicyclischen heterocyclischen Derivats mit der Formel I, oder eines pharmazeutisch geeigneten Salzes davon, nach einem der Ansprüche 1 bis 7 zur Herstellung eines neuen Arzneimittels zur Verwendung gegen durch Leukotriene hervorgerufene Erkrankungen oder medizinische Beschwerden.

## Patentansprüche (Patentansprüche für folgende(n) Vertragsstaat(en): GR, ES)

1. Verfahren zur Herstellung eines bicyclischen heterocyclischen Derivats mit der Formel I: 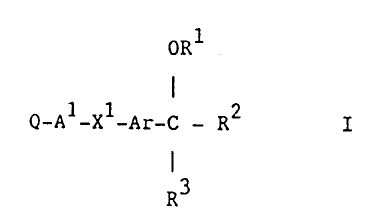 in der
Q für einen 9-gliedrigen bicyclischen heterocyclischen Rest steht, der ein oder zwei Stickstoff-Heteroatome und gegebenenfalls ein weiteres unter Stickstoff, Sauerstoff und Schwefel ausgewähltes Heteroatom enthält, wobei der heterocyclische Rest gegebenenfalls einen oder zwei Oxo- oder Thioxo-Substituenten und bis zu vier weitere Substituenten tragen kann, die unter Halogen, Hydroxy, Cyano, Amino, (1-4C)Alkyl, (1-4C)Alkoxy, Fluor-(1-4C)alkyl, (1-4C)Alkylamino, Di-[(1-4C)alkyl]amino, Amino-(1-4C)alkyl, (1-4C)Alkylamino-(1-4C)alkyl, Di-[(1-4C)alkyl]amino-(1-4C)alkyl, Phenyl und Phenyl-(1-4C)alkyl ausgewählt sind, wobei der Phenyl- oder der Phenyl-(1-4C)alkyl-Substituent gegebenenfalls einen Substituenten tragen kann, der unter Halogen, (1-4C)Alkyl und (1-4C)Alkoxy ausgewählt ist;
A¹ für eine Direktbindung zu X¹ oder für (1-3C)Alkylen steht;
X¹ für Oxy, Thio, Sulfinyl, Sulfonyl oder Imino steht; Ar für Phenylen steht, das gegebenenfalls einen oder zwei Substituenten tragen kann, die unter Halogen, Hydroxy, Amino, Nitro, Cyano, Carbamoyl, Ureido, (1-4C)Alkyl, (1-4C)Alkoxy, (1-4C)Alkylamino, Di-[(1-4C)alkyl]amino, Fluor-(1-4C)alkyl und (2-4C)Alkanoylamino ausgewählt sind; oder Ar für Pyridylen steht;
R¹ für (1-4C)Alkyl, (3-4C)Alkenyl oder (3-4C)Alkinyl steht; und
R² und R³ zusammen eine Gruppe mit der Formel -A²-X²-A³- bilden, die zusammen mit dem Kohlenstoffatom, an das A² und A³ gebunden sind, einen Ring mit 5 bis 7 Ringatomen definiert, wobei A² und A³, die gleich oder unterschiedlich sein können, jeweils für (1-3C)Alkylen stehen und X² für Oxy, Thio, Sulfinyl oder Sulfonyl steht, und wobei der Ring einen, zwei oder drei Substituenten tragen kann, die gleich oder unterschiedlich sein können, die unter Hydroxy, (1-4C)Alkyl und (1-4C)Alkoxy ausgewählt sind; oder in der
R¹ und R² zusammen eine Gruppe mit der Formel -A²-X²-A³- bilden, die zusammen mit dem Sauerstoffatom, an das A² gebunden ist, und mit dem Kohlenstoffatom, an das A³ gebunden ist, einen Ring mit 5 bis 7 Ringatomen definiert, wobei A² und A³, die gleich oder unterschiedlich sein können, jeweils für (1-3C)Alkylen stehen und X² für Oxy, Thio, Sulfinyl oder Sulfonyl steht, und wobei der Ring einen, zwei oder drei (1-4C)Alkyl-Substituenten tragen kann und R³ für (1-4C)Alkyl, (2-4C)Alkenyl oder (2-4C)Alkinyl steht;
oder eines pharmazeutisch geeigneten Salzes davon; dadurch **gekennzeichnet,** daß
(a) eine Verbindung mit der Formel Q-A¹-X¹-H mit einer Verbindung mit der Formel II, 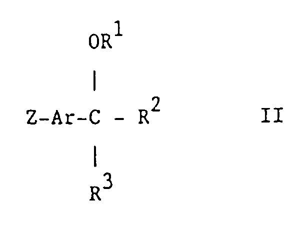 in der Z für eine austauschbare Gruppe steht, gekuppelt wird, und zwar mit der Maßgabe, daß jede Amino-, Alkylamino- oder Hydroxy-Gruppe mit einer üblichen Schutzgruppe geschützt sein kann, wenn eine Amino-, Alkylamino- oder Hydroxy-Gruppe in Q, Ar, R² oder R³ vorhanden ist, oder alternativ keine derartige Gruppe geschützt werden muß, woraufhin jede unerwünschte Schutzgruppe in Q, Ar, R² oder R³ mit üblichen Mitteln entfernt wird;
(b) eine Verbindung mit der Formel III 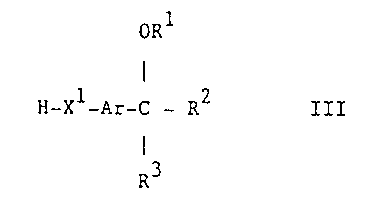 mit einer Verbindung mit der Formel Q-A¹-Z, in der Z für eine austauschbare Gruppe steht, gekuppelt wird, und zwar unter der Voraussetzung, daß jede Amino-, Alkylamino- oder Hydroxy-Gruppe mit einer üblichen Schutzgruppe geschützt sein kann, wenn eine Amino-, Alkylamino- oder Hydroxy-Gruppe in Q, Ar, R² oder R³ vorhanden ist, oder alternativ keine derartige Gruppe geschützt werden muß, woraufhin jede unerwünschte Schutzgruppe in Q, Ar, R² oder R³ mit üblichen Mitteln entfernt wird;
(c) eine Verbindung mit der Formel IV 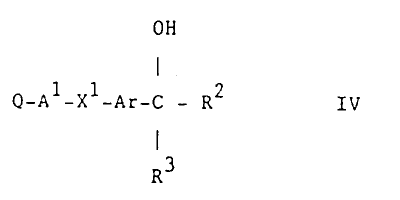 mit einer Verbindung mit der Formel R¹-Z, in der R¹ und Z die oben definierten Bedeutungen haben, alkyliert wird, und zwar unter der Voraussetzung, daß jede Amino-, Imino-, Alkylamino- oder Hydroxy-Gruppe mit einer üblichen Schutzgruppe geschützt sein kann, wenn eine Amino-, Imino-, Alkylamino- oder Hydroxy-Gruppe in Q, X¹, Ar, R² oder R³ vorhanden ist, oder alternativ keine derartige Gruppe geschützt werden muß, woraufhin jede unerwünschte Schutzgruppe in Q, X¹, Ar, R² oder R³ mit üblichen Mitteln entfernt wird;
(d) zur Herstellung derjenigen Verbindungen mit der Formel I, bei denen R¹ und R² zusammen eine Gruppe mit der Formel -A²-X²-A³- bilden, die zusammen mit dem Sauerstoffatom, an das A² gebunden ist, einen Ring mit 5 bis 7 Ringatomen definiert, wobei A², X² und A³ die oben definierten Bedeutungen haben, und wobei R³ die oben definierte Bedeutung hat, eine Verbindung mit der Formel V 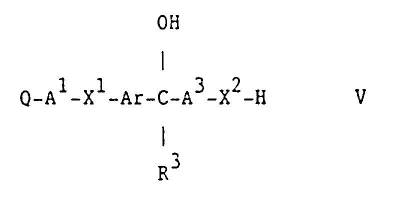 durch Umsetzung mit einem geeigneten Aldehyd oder Keton, oder mit einem Hemiacetal oder Acetal davon, oder mit einer Verbindung mit der Formel Z-A²-Z, in der Z die oben definierte Bedeutung hat, cyclisiert wird, und zwar unter der Voraussetzung, daß jede Amino-, Imino-, Alkylamino- oder Hydroxy-Gruppe mit einer üblichen Schutzgruppe geschützt ist, wenn eine Amino-, Imino-, Alkylamino- oder Hydroxy-Gruppe in Q, X¹ oder Ar vorhanden ist, woraufhin jede unerwünschte Schutzgruppe in Q, X¹ oder Ar mit üblichen Mitteln entfernt wird;
(e) zur Herstellung derjenigen Verbindungen mit der Formel I, bei denen X¹ für eine Sulfinyl- oder Sulfonyl-Gruppe steht, bei denen R² und R³ zusammen eine Gruppe mit der Formel -A²-X²-A³- bilden und X² für eine Sulfinyl- oder Sulfonyl-Gruppe steht, oder bei denen R¹ und R² zusammen eine Gruppe mit der Formel -A²-X²-A³- bilden und X² für eine Sulfinyl- oder Sulfonyl-Gruppe steht, eine Verbindung mit der Formel I, bei der X¹ für eine Thio-Gruppe steht, bei der R² und R³ zusammen eine Gruppe mit der Formel -A²-X²-A³- bilden und X² für eine Thio-Gruppe steht, oder bei der R¹ und R² zusammen eine Gruppe mit der Formel -A²-X²-A³- bilden und X² für eine Thio-Gruppe steht, oxidiert wird;
(f) zur Herstellung derjenigen Verbindungen mit der Formel I, bei denen Ar einen Alkanoylamino-Substituenten trägt, eine Verbindung mit der Formel I, in der Ar einen Amino-Substituenten trägt, acyliert wird;
(g) zur Herstellung derjenigen Verbindungen mit der Formel I, bei denen Q einen Alkyl- oder substituierten Alkyl-Substituenten an einem verfügbaren Stickstoffatom trägt, oder bei denen Ar einen Alkoxy-Substituenten trägt, eine Verbindung mit Formel I, bei der Q ein Wasserstoffatom an dem verfügbaren Stickstoffatom trägt, oder bei der Ar einen Hydroxy-Substituenten trägt, alkyliert wird; und
wenn ein pharmazeutisch geeignetes Salz einer neuen Verbindung mit der Formel I benötigt wird, dieses durch Umsetzung der Verbindung mit einer geeigneten Säure oder Base unter Anwendung eines üblichen Verfahrens erhältlich ist.

2. Verfahren nach Anspruch 1, zur Herstellung eines bicyclischen heterocyclischen Derivats mit der Formel I, in der
Q Indolyl, Indolinyl, Benzimidazolyl, 2,3-Dihydrobenzimidazolyl, 1H-Indazolyl, 2,3-Dihydro-1H-indazolyl, Benzoxazolyl, 2,3-Dihydrobenzoxazolyl, Benzothiazolyl oder 2,3-Dihydrobenzothiazolyl steht, das gegebenenfalls einen oder zwei Oxo- oder Thioxo-Substituenten und bis zu vier Substituenten tragen kann, die unter Fluor, Chlor, Brom, Hydroxy, Cyano, Amino, Methyl, Ethyl, Propyl, Methoxy, Trifluormethyl, 2-Fluorethyl, 2,2,2-Trifluorethyl, 2-Methylaminoethyl, 2-Dimethylaminoethyl, Phenyl und Benzyl ausgewählt sind, wobei der Phenyl- oder Benzyl-Substituent gegebenenfalls einen Substituenten tragen kann, der unter Chlor, Methyl und Methoxy ausgewählt ist; A¹ für eine Direktbindung zu X¹ oder für Methylen steht;
X¹ für Oxy, Thio, Sulfinyl oder Sulfonyl steht; Ar für 1,3-Phenylen oder 1,4-Phenylen steht, das gegebenenfalls einen oder zwei Substituenten tragen kann, die unter Fluor, Chlor, Hydroxy, Amino, Nitro, Ureido, Methoxy, Dimethylamino, Trifluormethyl und Acetamido ausgewählt sind; oder in der Ar für 3,5-Pyridylen steht;
R¹ für Methyl, Ethyl, Allyl oder 2-Propinyl steht; und
R² und R³ zusammen eine Gruppe mit der Formel -A²-X²-A³- bilden, die zusammen mit dem Kohlenstoffatom, an das A² und A³ gebunden sind, einen Ring mit 5 oder 6 Ringatomen definiert, wobei A² für Ethylen steht, A³ für Methylen oder Ethylen steht und X² für Oxy steht, und wobei der Ring einen oder zwei Substituenten tragen kann, die unter Methyl, Ethyl, Propyl, Methoxy und Ethoxy ausgewählt sind; oder in der
R¹ und R² zusammen eine Gruppe mit der Formel -A²-X²-A³- bilden, die zusammen mit dem Sauerstoffatom, an das A² gebunden ist, und mit dem Kohlenstoffatom, an das A³ gebunden ist, einen Ring mit 5 oder 6 Ringatomen definiert, wobei A² für Methylen steht, A³ für Methylen oder Ethylen steht und X² für Oxy steht, und wobei der Ring einen, zwei oder drei Substituenten tragen kann, die unter Methyl, Ethyl und Propyl ausgewählt sind, und R³ für Methyl oder Ethyl steht; oder eines pharmazeutisch geeigneten Salzes davon.

3. Verfahren nach Anspruch 1 zur Herstellung eines bicyclischen heterocyclischen Derivats mit der Formel I, in der
Q für 2-Oxoindolin-5-yl, 2,3-Dioxoindolin-5-yl, 2-Oxo-2,3-dihydrobenzimidazol-5-yl, 2-Oxo-2,3-dihydrobenzoxazol-6-yl oder 2-Oxo-2,3-dihydrobenzothiazol-6-yl steht, das gegebenenfalls einen, zwei oder drei Substituenten tragen kann, die unter Fluor, Chlor, Hydroxy, Methyl, Ethyl, Propyl, 2-Fluorethyl, 2-Dimethylaminoethyl, Phenyl und Benzyl ausgewählt sind;
A¹ für eine Direktbindung zu X¹ oder für Methylen steht;
X¹ für Oxy, Thio, Sulfinyl oder Sulfonyl steht;
Ar für 1,3-Phenylen oder 1,4-Phenylen steht, das gegebenenfalls einen oder zwei Substituenten tragen kann, die unter Fluor, Hydroxy, Amino, Nitro, Ureido, Methoxy, Dimethylamino, Trifluormethyl und Acetamido ausgewählt sind; oder in der
Ar für 3,5-Pyridylen steht;
R¹ für Methyl, Ethyl, Allyl oder 2-Propinyl steht; und
R² und R³ zusammen eine Gruppe mit der Formel -A²-X²-A³- bilden, die zusammen mit dem Kohlenstoffatom, an das A² und A³ gebunden sind, einen Ring mit 5 oder 6 Ringatomen definiert, wobei A² für Ethylen steht, A³ für Methylen oder Ethylen steht und
X² für Oxy steht, und wobei der Ring einen oder zwei Substituenten tragen kann, die unter Methyl und Ethyl ausgewählt sind; oder in der
R¹ und R² zusammen eine Gruppe mit der Formel -A²-X²-A³- bilden, die zusammen mit dem Sauerstoffatom, an das A² gebunden ist, und mit dem Kohlenstoffatom, an das A³ gebunden ist, einen Ring mit 5 Ringatomen definiert, wobei A² für Methylen steht, A³ für Methylen steht und X² für Oxy steht, und wobei der Ring einen, zwei oder drei Substituenten tragen kann, die unter Methyl und Ethyl ausgewählt sind, und R³ für Methyl oder Ethyl steht; oder eines pharmazeutisch geeigneten Salzes davon.

4. Verfahren nach Anspruch 1 zur Herstellung eines bicyclischen heterocyclischen Derivats mit der Formel I, in der
Q für 1,3,3-Trimethyl-2-oxoindolin-5-yl, 1,3-Dimethyl-2-oxo-2,3-dihydrobenzimidazol-5-yl oder 3-Methyl-2-oxo-2,3-dihydrobenzothiazol-6-yl steht;
A¹ für eine Direktbindung zu X¹ steht;
X¹ für Thio steht;
Ar für 1,3-Phenylen oder 5-Fluor-1,3-phenylen steht;
R¹ für Methyl steht;
R² und R³ zusammen eine Gruppe mit der Formel -A²-X²-A³- bilden, die zusammen mit dem Kohlenstoffatom, an das A² und A³ gebunden sind, einen Ring mit 6 Ringatomen definiert, wobei A² und A³ jeweils für Ethylen stehen und X² für Oxy steht, und wobei der Ring einen Methyl- oder Ethyl-Substituenten alpha zu X² tragen kann; oder eines pharmazeutisch geeigneten Salzes davon.

5. Verfahren nach Anspruch 1 zur Herstellung eines bicyclischen heterocyclischen Derivats mit der Formel I, in der
Q für 1,3,3-Trimethyl-2-oxoindolin-5-yl, 1,3-Dimethyl-2-oxo-2,3-dihydrobenzimidazol-5-yl, 3-Methyl-2-oxo-2,3-dihydrobenzothiazol-6-yl oder 3,3-Difluor-1-methyl-2-oxoindolin-5-yl steht;
A¹ für eine Direktbindung zu X¹ steht;
X¹ für Thio steht;
Ar für 1,3-Phenylen oder 5-Fluor-1,3-phenylen steht;
R¹ für Methyl steht;
R² und R³ zusammen eine Gruppe mit der Formel -A²-X²-A³- bilden, die zusammen mit dem Kohlenstoffatom, an das A² und A³ gebunden sind, einen Ring mit 6 Ringatomen definiert, wobei A² und A³ jeweils für Ethylen stehen und X² für Oxy steht, und wobei der Ring einen Methyl- oder Ethyl-Substituenten alpha zu X² tragen kann; oder eines pharmazeutisch geeigneten Salzes davon.

6. Verfahren zur Herstellung einer pharmazeutischen Zusammensetzung, dadurch **gekennzeichnet,** daß ein bicyclisches heterocyclisches Derivat mit der Formel I, oder ein pharmazeutisch geeignetes Salz davon, wie in einem der Ansprüche 1 bis 5 definiert, und ein pharmazeutisch geeignetes Streckmittel oder Trägermittel miteinander in Verbindung gebracht werden.

## Revendications (Revendications pour l'(les) Etat(s) contractant(s) suivant(s): AT, BE, CH, DE, DK, FR, GB, IT, LI, LU, NL, SE)

1. Dérivé hétérocyclique bicyclique de formule I 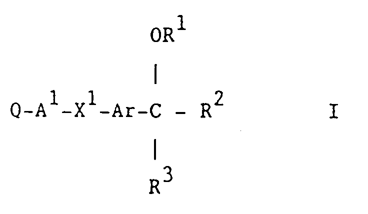 dans laquelle Q est un groupement hétérocyclique bicyclique à 9 chaînons contenant un ou deux hetéroatomes d'azote et contenant facultativement un autre hétéroatome choisi entre l'azote, l'oxygène et le soufre, ce groupement hétérocyclique pouvant facultativement porter un ou deux substituants oxo ou thioxo et jusqu'à quatre autres substituants choisis entre des substituants halogéno, hydroxy, cyano, amino, alkyle en C₁ à C₄, alkoxy en C₁ à C₄, fluoralkyle en C₁ à C₄, (alkyle en C₁ à C₄)-amino, di(alkyle en C₁ à C₄)-amino, amino-(alkyle en C₁ à C₄), (alkyle en C₁ à C₄)-amino-(alkyle en C₁ à C₄), di(alkyle en C₁ à C₄)-amino-(alkyle en C₁ à C₄), phényle et phényl-(alkyle en C₁ à C₄), le substituant phényle ou phényl(alkyle en C₁ à C₄) pouvant facultativement porter un substituant choisi entre halogéno, alkyle en C₁ à C₄ et alkoxy en C₁ à C₄,
A¹ est une liaison directe à X¹ ou représente un groupe alkylène en C₁ à C₃ ;
X¹ est un groupe oxy, thio, sulfinyle, sulfonyle ou imino ;
Ar est un groupe phénylène qui peut facuitativement porter un ou deux substituants choisis entre des substituants halogéno, hydroxy, amino, nitro, cyano, carbamoyle, uréido, alkyle en C₁ à C₄, alkoxy en C₁ à C₄, (alkyle en C₁ à C₄)-amino, di(alkyle en C₁ à C₄)-amino, fluoralkyle en C₁ à C₄ et (alcanoyle en C₂ à C₄)-amino ; ou bien
Ar est un groupe pyridylène ;
R¹ est un groupe alkyle en C₁ à C₄, alcényle en C₃ ou C₄ ou alcynyle en C₃ ou C₄, et
R² et R³ forment ensemble un groupe de formule -A²-X²-A³- qui, conjointement avec l'atome de carbone auquel A² et A³ sont attachés, définit un noyau pentagonal à heptagonal, formule dans laquelle A² et A³, qui peuvent être identiques ou différents, représentent chacun un groupe alkylène en C₁ à C₃ et X² est un groupe oxy, thio, sulfinyle ou sulfonyle, ce noyau pouvant porter un, deux ou trois substituants, identiques ou différents, choisis entre des substituants hydroxy, alkyle en C₁ à C₄ et alkoxy en C₁ à C₄ ;
ou bien R¹ et R² forment ensemble un groupe de formule -A²-X²-A³- qui, conjointement avec l'atome d'oxygène auquel A² est attaché et avec l'atome de carbone auquel A³ est attaché, définit un noyau pentagonal à heptagonal, formule dans laquelle A² et A³, qui peuvent être identiques ou différents, représentent chacun un groupe alkylène en C₁ à C₃ et X² est un groupe oxy, thio, sulfinyle ou sulfonyle, ce noyau pouvant porter un, deux ou trois substituants alkyle en C₁ à C₄, et R³ est un groupe alkyle en C₁ à C₄, alcényle en C₂ à C₄ ou alcynyle en C₂ à C₄ ;
ou un sel pharmaceutiquement acceptable de ce dérivé.

2. Dérivé hétérocyclique bicyclique de formule I suivant la revendication 1,
dans lequel Q est un groupe indolyle, indolinyle, benzimidazolyle, 2,3-dihydrobenzimidazolyle, 1H-indazolyle, 2,3-dihydro-1H-indazolyle, benzoxazolyle, 2,3-dihydrobenzoxazolyle, benzothiazolyle ou 2,3-dihydrobenzothiazolyle qui peut facultativement porter un ou deux substituants oxo ou thioxo et jusqu'à quatre substituants choisis entre des substituants fluoro, chloro, bromo, hydroxy, cyano, amino, méthyle, éthyle, propyle, méthoxy, trifluorométhyle, 2-fluoréthyle, 2,2,2-trifluoréthyle, 2-méthylaminoéthyle, 2-diméthylaminoéthyle, phényle et benzyle, et le substituant phényle ou benzyle pouvant facultativement porter un substituant choisi entre chloro, méthyle et méthoxy ;
A¹ est une liaison directe à X¹ ou représente un groupe méthylène ;
X¹ est un groupe oxy, thio, sulfinyle ou sulfonyle ;
Ar est un groupe 1,3-phénylène ou 1,4-phénylène qui peut facultativement porter un ou deux substituants choisis entre des substituants fluoro, chloro, hydroxy, amino, nitro, uréido, méthoxy, diméthylamino, trifluorométhyle et acétamido ; ou bien
Ar est un groupe 3,5-pyridylène ;
R¹ est groupe méthyle, éthyle, allyle ou 2-propynyle ; et
R² et R³ forment ensemble un groupe de formule -A²-X²-A³- qui, conjointement avec l'atome de carbone auquel A² et A³ sont attachés, définit un noyau pentagonal ou hexagonal, formule dans laquelle A² est un groupe éthylène, A³ est un groupe méthylène ou éthylène et X² est un groupe oxy, ce noyau pouvant porter un ou deux substituants choisis entre méthyle, éthyle, propyle, méthoxy et éthoxy ;
ou bien R¹ et R² forment ensemble un groupe de formule -A²-X²-A³- qui, conjointement avec l'atome d'oxygène auquel A² est attaché et avec l'atome de carbone auquel A³ est attaché, définit un noyau pentagonal ou hexagonal, formule dans laquelle A² est un groupe méthylène, A³ est un groupe méthylène ou éthylène et X² est un groupe oxy, et ce noyau pouvant porter un, deux ou trois substituants choisis entre méthyle, éthyle et propyle, et R³ est un groupe méthyle ou éthyle ;
ou un sel pharmaceutiquement acceptable de ce dérivé.

3. Dérivé hétérocyclique bicyclique de formule I suivant la revendication 1,
dans lequel Q est un groupe 2-oxoindoline-5-yle, 2,3-dioxoindoline-5-yle, 2-oxo-2,3-dihydrobenzimidazole-5-yle, 2-oxo-2,3-dihydrobenzoxazole-6-yle ou 2-oxo-2,3-dihydrobenzothiazole-6-yle qui peut facultativement porter un, deux ou trois substituants choisis entre des substituants fluoro, chloro, hydroxy, méthyle, éthyle, propyle, 2-fluoréthyle, 2-diméthylaminoéthyle, phényle et benzyle ;
A¹ est une liaison directe à X¹ ou représente un groupe méthylène ;
X¹ est un groupe oxy, thio, sulfinyle ou sulfonyle ;
Ar est un groupe 1,3-phénylène ou 1,4-phénylène qui peut facultativement porter un ou deux substituants choisis entre des substituants fluoro, hydroxy, amino, nitro, uréido, méthoxy, diméthylamino, trifluorométhyle et acétamido ; ou bien
Ar est un groupe 3,5-pyridylène ;
R¹ est un groupe méthyle, éthyle, allyle ou 2-propynyle ; et
R² et R³ forment conjointement un groupe de formule -A²-X²-A³- qui, conjointement avec l'atome de carbone auquel A² et A³ sont attachés, définit un noyau pentagonal ou hexagonal, formule dans laquelle A² est un groupe éthylène, A³ est un groupe méthylène ou éthylène et X² est un groupe oxy, ce noyau pouvant porter un ou deux substituants choisis entre méthyle et éthyle ;
ou bien R¹ et R² forment ensemble un groupe de formule -A²-X²-A³- qui, conjointement avec l'atome d'oxygène auquel A² est attaché et avec l'atome de carbone auquel A³ est attaché, définit un noyau pentagonal, formule dans laquelle A² est un groupe méthylène, A³ est un groupe méthylène et X² est un groupe oxy, ce noyau pouvant porter un, deux ou trois substituants choisis entre méthyle et éthyle, et R³ est un groupe méthyle ou éthyle ;
ou un sel pharmaceutiquement acceptable de ce dérivé.

4. Dérivé hétérocyclique bicyclique de formule I suivant la revendication 1,
dans lequel Q est un groupe 1,3,3-triméthyl-2-oxoindoline-5-yle, 1,3-diméthyl-2-oxo-2,3-dihydrobenzimidazole-5-yle ou 3-méthyl-2-oxo-2,3-dihydrobenzothiazole-6-yle ;
A¹ est une liaison directe à X¹ ;
X¹ est un groupe thio ;
Ar est un groupe 1,3-phénylène ou 5-fluoro-1,3-phénylène ;
R¹ est un groupe méthyle ; et
R² et R³ forment ensemble un groupe de formule -A²-X²-A³- qui, conjointement avec l'atome de carbone auquel A² et A³ sont attachés, définit un noyau hexagonal, formule dans laquelle chacun de A² et A³ représente un groupe éthylène et X² est un groupe oxy et ce noyau pouvant porter un substituant méthyle ou éthyle en alpha par rapport à X² ;
ou un sel pharmaceutiquement acceptable de ce dérivé.

5. Dérivé hétérocyclique bicyclique de formule I suivant la revendication 1,
dans lequel Q est un groupe 1,3,3-triméthyl-2-oxoindoline-5-yle, 1,3-diméthyl-2-oxo-2,3-dihydrobenzimidazole-5-yle, 3-méthyl-2-oxo-2,3-dihydrobenzothiazole-6-yle ou 3,3-difluoro-1-méthyl-2-oxoindoline-5-yle ;
A¹ est une liaison directe à X¹ ;
X¹ est un groupe thio ;
Ar est un groupe 1,3-phénylène ou 5-fluoro-1,3-phénylène ;
R¹ est un groupe méthyle et
R² et R³ forment ensemble un groupe de formule -A²-X²-A³- qui, conjointement avec l'atome de carbone auquel A² et A³ sont attachés, définit un noyau hexagonal, formule dans laquelle chacun de A² et A³ est un groupe éthylène et X² est un groupe oxy, et le noyau pouvant porter un substituant méthyle ou éthyle en alpha par rapport à X² ;
ou un sel pharmaceutiquement acceptable de ce dérivé.

6. Dérivé hétérocyclique bicyclique de formule I ou un sel pharmaceutiquement acceptable de ce dérivé tel que défini dans la revendication 1, choisi dans le groupe consistant en :
le 4-méthoxy-4-[3-(1,3,3-triméthyl-2-oxoindoline-5-ylthio)phényl]tétrahydropyranne,
le 4-[3-(1,3-diméthyl-2-oxo-2,3-dihydrobenzimidazole-5-ylthio)-phényl]-4-méthoxytétrahydropyranne et le 4-méthoxy-4-[3-(3-méthyl-2-oxo-2,3-dihydrobenzothiazole-6-ylthio)phényl]tétrahydropyranne.

7. Dérivé hétérocyclique bicyclique de formule I ou un sel pharmaceutiquement acceptable de ce dérivé tel que défini dans la revendication 1, choisi dans le groupe consistant en :
le 4-[5-fluoro-3-(3-méthyl-2-oxo-2,3-dihydrobenzothiazole-6-ylméthoxy)phényl]-4-méthyltétrahydropyranne,
le 4-[5-fluoro-3-(1,3-diméthyl-2-oxo(2,3-dihydrobenzimidazole-5-ylméthoxy)phényl]-4-méthoxytétrahydropyranne et
le 4-[5-fluoro-3-(3,3-difluoro-1-méthyl-2-oxoindoline-5-ylméthoxy)phényl]-4-méthoxytétrahydropyranne.

8. Procédé de production du dérivé hétérocyclique bicyclique de formule I ou d'un sel pharmaceutiquement acceptable de ce dérivé suivant la revendication 1, qui comprend :
(a) le couplage d'un composé de formule Q-A¹-X¹-H avec un composé de formule II 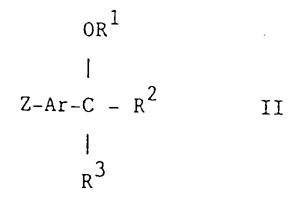 dans laquelle Z est un groupe déplaçable, sous réserve que, lorsque Q, Ar, R² ou R³ renferment un groupe amino, alkylamino ou hydroxy, tout groupe amino, alkylamino ou hydroxy puisse être protégé par un groupe protecteur classique ou bien, en variante, tout groupe tel qu'indiqué ne nécessite pas d'être protégé ; après quoi, tout groupe protecteur non désiré présent dans Q, Ar, R² ou R³ est éliminé par des moyens classiques ;
(b) le couplage d'un composé de formule III 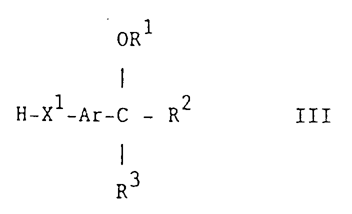 avec un composé de formule Q-A¹-Z dans laquelle Z est un groupe déplaçable ; sous réserve que lorsqu'un groupe amino, alkylamino ou hydroxy est présent dans Q, Ar, R² ou R³, tout groupe amino, alkylamino ou hydroxy puisse être protégé par un groupe protecteur classique ou bien, en variante, tout groupe tel qu'indiqué ne nécessite pas d'être protégé, après quoi tout groupe protecteur non désiré présent Q, Ar, R² ou R³ est éliminé par des moyens classiques ;
(c) l'alkylation d'un composé de formule IV 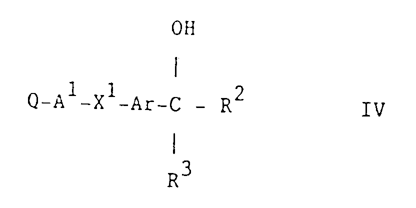 avec un composé de formule R¹-Z, dans laquelle R¹ et Z ont les définitions indiquées ci-dessus ; sous réserve que lorsque Q, X¹, Ar, R² ou R³ renferme un groupe amino, imino, alkylamino ou hydroxy, tout groupe amino, imino, alkylamino ou hydroxy puisse être protégé par un groupe protecteur classique ou bien, en variante, tout groupe tel que mentionné ne nécessite pas d'être protégé, après quoi tout groupe protecteur non désiré présent dans Q, X¹, Ar, R² ou R³ est éliminé par des moyens classiques ;
(d) pour la production de composés de formule I dans laquelle R¹ et R² forment ensemble un groupe de formule -A²-X²-A³- qui, conjointement avec l'atome d'oxygène auquel A² est attaché, définit un noyau pentagonal à heptagonal, A², X² et A³ ayant les définitions données ci-dessus, et dans laquelle R³ a la définition indiquée ci-dessus ; la cyclisation d'un composé de formule V 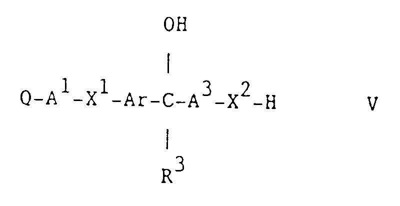 par réaction avec un aldéhyde ou une cétone approprié ou avec son hémiacétal ou son acétal ou avec un composé de formule Z-A²-Z dans laquelle Z a la définition indiquée ci-dessus, sous réserve que lorsque Q, X¹ ou Ar contient un groupe amino, imino, alkylamino ou hydroxy, tout groupe amino, imino, alkylamino ou hydroxy soit protégé par un groupe protecteur classique, après quoi tout groupe protecteur non désiré dans Q, X¹ ou Ar est éliminé par des moyens classiques ;
(e) pour la production des composés de formule I dans laquelle X¹ est un groupe sulfinyle ou sulfonyle, R² et R³ forment ensemble un groupe de formule -A²-X²-A³- et X² est un groupe sulfinyle ou sulfonyle, ou dans laquelle R¹ et R² forment ensemble un groupe de formule -A²-X²-A³- et X² est un groupe sulfinyle ou sulfonyle, l'oxydation d'un composé de formule I dans laquelle X¹ est un groupe thio, R² et R³ forment un groupe de formule -A²-X²-A³- et X² est un groupe thio, ou bien R¹ et R² forment ensemble un groupe de formule -A²-X²-A³- et X² est un groupe thio ;
(f) pour la production des composés de formule I dans laquelle Ar porte un substituant alcanoylamino, l'acylation d'un composé de formule I dans laquelle Ar porte un substituant amino ; ou bien
(g) pour la production des composés de formule I dans laquelle Q porte sur un atome d'azote disponible un substituant alkyle ou alkyle substitué, ou dans laquelle Ar porte un substituant alkoxy, l'alkylation d'un composé de formule I dans laquelle Q porte un atome d'hydrogène sur l'atome d'azote disponible en question, ou dans laquelle Ar porte un substituant hydroxy ; et
lorsqu'on désire obtenir un sel pharmaceutiquement acceptable d'un composé nouveau de formule I, on peut l'obtenir par réaction de ce composé avec un acide ou une base convenable par un mode opératoire classique.

9. Composition pharmaceutique, qui comprend un dérivé hétérocyclique bicyclique de formule I ou un sel pharmaceutiquement acceptable de ce dérivé, suivant l'une quelconque des revendications 1 à 7, en association avec un diluant ou support acceptable du point de vue pharmaceutique.

10. Utilisation d'un dérivé hétérocyclique bicyclique de formule I ou d'un sel pharmaceutiquement acceptable de ce dérivé suivant l'une quelconque des revendications 1 à 7 dans la préparation d'un médicament nouveau destiné à être utilisé dans une maladie ou un état pathologique dont le médiateur est un leucotriène.

## Revendications (Revendications pour l'(les) Etat(s) contractant(s) suivant(s): GR, ES)

1. Procédé de production d'un dérivé hétérocyclique bicyclique de formule I 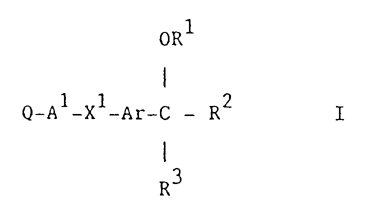 dans laquelle Q est un groupement hétérocyclique bicyclique à 9 chaînons contenant un ou deux hétéroatomes d'azote et contenant facultativement un autre hétéroatome choisi entre l'azote, l'oxygène et le soufre, ce groupement hétérocyclique pouvant facultativement porter un ou deux substituants oxo ou thioxo et jusqu'à quatre autres substituants choisis entre des substituants halogéno, hydroxy, cyano, amino, alkyle en C₁ à C₄, alkoxy en C₁ à C₄, fluoralkyle en C₁ à C₄, (alkyle en C₁ à C₄)-amino, di(alkyle en C₁ à C₄)-amino, amino-(alkyle en C₁ à C₄), (alkyle en C₁ à C₄)-amino-(alkyle en C₁ à C₄), di(alkyle en C₁ à C₄)-amino-(alkyle en C₁ à C₄), phényle et phényl-(alkyle en C₁ à C₄), le substituant phényle ou phényl(alkyle en C₁ à C₄) pouvant facultativement porter un substituant choisi entre halogéno, alkyle en C₁ à C₄ et alkoxy en C₁ à C₄,
A¹ est une liaison directe à X¹ ou représente un groupe alkylène en C₁ à C₃ ;
X¹ est un groupe oxy, thio, sulfinyle, sulfonyle ou imino ;
Ar est un groupe phénylène qui peut facultativement porter un ou deux substituants choisis entre des substituants halogéno, hydroxy, amino, nitro, cyano, carbamoyle, uréido, alkyle en C₁ à C₄, alkoxy en C₁ à C₄, (alkyle en C₁ à C₄)-amino, di(alkyle en C₁ à C₄)-amino, fluoralkyle en C₁ à C₄ et (alcanoyle en C₂ à C₄)-amino ; ou bien
Ar est un groupe pyridylène ;
R¹ est un groupe alkyle en C₁ à C₄, alcényle en C₃ ou C₄ ou alcynyle en C₃ ou C₄, et
R² et R³ forment ensemble un groupe de formule -A²-X²-A³- qui, conjointement avec l'atome de carbone auquel A² et A³ sont attachés, définit un noyau pentagonal à heptagonal, formule dans laquelle A² et A³, qui peuvent être identiques ou différents, représentent chacun un groupe alkylène en C₁ à C₃ et X² est un groupe oxy, thio, sulfinyle ou sulfonyle, ce noyau pouvant porter un, deux ou trois substituants, identiques ou différents, choisis entre des substituants hydroxy, alkyle en C₁ à C₄ et alkoxy en C₁ à C₄ ;
ou bien R¹ et R² forment ensemble un groupe de formule -A²-X²-A³- qui, conjointement avec l'atome d'oxygène auquel A² est attaché et avec l'atome de carbone auquel A³ est attaché, définit un noyau pentagonal à heptagonal, formule dans laquelle A² et A³, qui peuvent être identiques ou différents, représentent chacun un groupe alkylène en C₁ à C₃ et X² est un groupe oxy, thio, sulfinyle ou sulfonyle, ce noyau pouvant porter un, deux ou trois substituants alkyle en C₁ à C₄, et R³ est un groupe alkyle en C₁ à C₄, alcényle en C₂ à C₄ ou alcynyle en C₂ à C₄ ;
ou d'un sel pharmaceutiquement acceptable de ce dérivé ;
caractérisé par
(a) le couplage d'un composé de formule Q-A¹-X¹-H avec un composé de formule II 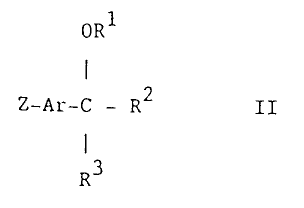 dans laquelle Z est un groupe déplaçable, sous réserve que, lorsque Q, Ar, R² ou R³ renferment un groupe amino, alkylamino ou hydroxy, tout groupe amino, alkylamino ou hydroxy puisse être protégé par un groupe protecteur classique ou bien, en variante, tout groupe tel qu'indiqué ne nécessite pas d'être protégé ; après quoi, tout groupe protecteur non désiré présent dans Q, Ar, R² ou R³ est éliminé par des moyens classiques ;
(b) le couplage d'un composé de formule III 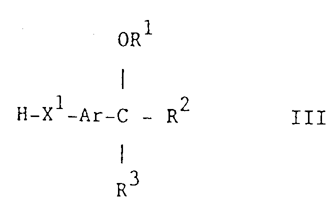 avec un composé de formule Q-A¹-Z dans laquelle Z est un groupe déplaçable ; sous réserve que lorsqu'un groupe amino, alkylamino ou hydroxy est présent dans Q, Ar, R² ou R³, tout groupe amino, alkylamino ou hydroxy puisse être protégé par un groupe protecteur classique ou bien, en variante, tout groupe tel qu'indiqué ne nécessite pas d'être protégé, après quoi tout groupe protecteur non désiré présent Q, Ar, R² ou R³ est éliminé par des moyens classiques ;
(c) l'alkylation d'un composé de formule IV 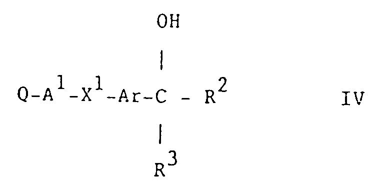 avec un composé de formule R¹-Z, dans laquelle R¹ et Z ont les définitions indiquées ci-dessus ; sous réserve que lorsque Q, X¹, Ar, R² ou R³ renferme un groupe amino, imino, alkylamino ou hydroxy, tout groupe amino, imino, alkylamino ou hydroxy puisse être protégé par un groupe protecteur classique ou bien, en variante, tout groupe tel que mentionné ne nécessite pas d'être protégé, après quoi tout groupe protecteur non désiré présent dans Q, X¹, Ar, R² ou R³ est éliminé par des moyens classiques ;
(d) pour la production de composés de formule I dans laquelle R¹ et R² forment ensemble un groupe de formule -A²-X²-A³- qui, conjointement avec l'atome d'oxygène auquel A² est attaché, définit un noyau pentagonal à heptagonal, A², X² et A³ ayant les définitions données ci-dessus, et dans laquelle R³ a la définition indiquée ci-dessus ; la cyclisation d'un composé de formule V 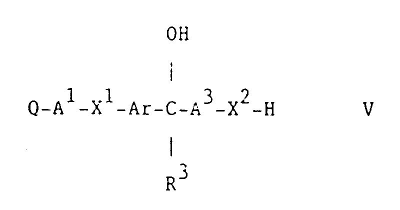 par réaction avec un aldéhyde ou une cétone approprié ou avec son hémiacétal ou son acétal ou avec un composé de formule Z-A²-Z dans laquelle Z a la définition indiquée ci-dessus, sous réserve que lorsque Q, X¹ ou Ar contient un groupe amino, imino, alkylamino ou hydroxy, tout groupe amino, imino, alkylamino ou hydroxy soit protégé par un groupe protecteur classique, après quoi tout groupe protecteur non désiré dans Q, X¹ ou Ar est éliminé par des moyens classiques ;
(e) pour la production des composés de formule I dans laquelle X¹ est un groupe sulfinyle ou sulfonyle, R² et R³ forment ensemble un groupe de formule -A²-X²-A³- et X² est un groupe sulfinyle ou sulfonyle, ou dans laquelle R¹ et R² forment ensemble un groupe de formule -A²-X²-A³- et X² est un groupe sulfinyle ou sulfonyle, l'oxydation d'un composé de formule I dans laquelle X¹ est un groupe thio, R² et R³ forment un groupe de formule -A²-X²-A³- et X² est un groupe thio, ou bien R¹ et R² forment ensemble un groupe de formule -A²-X²-A³- et X² est un groupe thio ;
(f) pour la production des composés de formule I dans laquelle Ar porte un substituant alcanoylamino, l'acylation d'un composé de formule I dans laquelle Ar porte un substituant amino ; ou bien
(g) pour la production des composés de formule I dans laquelle Q porte sur un atome d'azote disponible un substituant alkyle ou alkyle substitué, ou dans laquelle Ar porte un substituant alkoxy, l'alkylation d'un composé de formule I dans laquelle Q porte un atome d'hydrogène sur l'atome d'azote disponible en question, ou dans laquelle Ar porte un substituant hydroxy ; et
lorsqu'on désire obtenir un sel pharmaceutiquement acceptable d'un composé nouveau de formule I, on peut l'obtenir par réaction de ce composé avec un acide ou une base convenable par un mode opératoire classique.

2. Procédé suivant la revendication 1, pour la production d'un dérivé hétérocyclique bicyclique de formule I dans laquelle Q est un groupe indolyle, indolinyle, benzimidazolyle, 2,3-dihydrobenzimidazolyle, 1H-indazolyle, 2,3-dihydro-1H-indazolyle, benzoxazolyle, 2,3-dihydrobenzoxazolyle, benzothiazolyle ou 2,3-dihydrobenzothiazolyle qui peut facultativement porter un ou deux substituants oxo ou thioxo et jusqu'à quatre substituants choisis entre des substituants fluoro, chloro, bromo, hydroxy, cyano, amino, méthyle, éthyle, propyle, méthoxy, trifluorométhyle, 2-fluoréthyle, 2,2,2-trifluoréthyle, 2-méthylaminoéthyle, 2-diméthylaminoéthyle, phényle et benzyle, et le substituant phényle ou benzyle pouvant facultativement porter un substituant choisi entre chloro, méthyle et méthoxy ;
A¹ est une liaison directe à X¹ ou représente un groupe méthylène ;
X¹ est un groupe oxy, thio, sulfinyle ou sulfonyle ;
Ar est un groupe 1,3-phénylène ou 1,4-phénylène qui peut facultativement porter un ou deux substituants choisis entre des substituants fluoro, chloro, hydroxy, amino, nitro, uréido, méthoxy, diméthylamino, trifluorométhyle et acétamido ; ou bien
Ar est un groupe 3,5-pyridylène ;
R¹ est groupe méthyle, éthyle, allyle ou 2-propynyle ; et
R² et R³ forment ensemble un groupe de formule -A²-X²-A³- qui, conjointement avec l'atome de carbone auquel A² et A³ sont attachés, définit un noyau pentagonal ou hexagonal, formule dans laquelle A² est un groupe éthylène, A³ est un groupe méthylène ou éthylène et X² est un groupe oxy, ce noyau pouvant porter un ou deux substituants choisis entre méthyle, éthyle, propyle, méthoxy et éthoxy ;
ou bien R¹ et R² forment ensemble un groupe de formule -A²-X²-A³- qui, conjointement avec l'atome d'oxygène auquel A² est attaché et avec l'atome de carbone auquel A³ est attaché, définit un noyau pentagonal ou hexagonal, formule dans laquelle A² est un groupe méthylène, A³ est un groupe méthylène ou éthylène et X² est un groupe oxy, et ce noyau pouvant porter un, deux ou trois substituants choisis entre méthyle, éthyle et propyle, et R³ est un groupe méthyle ou éthyle ;
ou d'un sel pharmaceutiquement acceptable de ce dérivé.

3. Procédé suivant la revendication 1, pour la production d'un dérivé hétérocyclique bicyclique de formule I dans lequel Q est un groupe 2-oxoindoline-5-yle, 2,3-dioxoindoline-5-yle, 2-oxo-2,3-dihydrobenzimidazole-5-yle, 2-oxo-2,3-dihydrobenzoxazole-6-yle ou 2-oxo-2,3-dihydrobenzothiazole-6-yle qui peut facultativement porter un, deux ou trois substituants choisis entre des substituants fluoro, chloro, hydroxy, méthyle, éthyle, propyle, 2-fluoréthyle, 2-diméthylaminoéthyle, phényle et benzyle ;
A¹ est une liaison directe à X¹ ou représente un groupe méthylène ;
X¹ est un groupe oxy, thio, sulfinyle ou sulfonyle ;
Ar est un groupe 1,3-phénylène ou 1,4-phénylène qui peut facultativement porter un ou deux substituants choisis entre des substituants fluoro, hydroxy, amino, nitro, uréido, méthoxy, diméthylamino, trifluorométhyle et acétamido ; ou bien
Ar est un groupe 3,5-pyridylène ;
R¹ est un groupe méthyle, éthyle, allyle ou 2-propynyle ; et
R² et R³ forment conjointement un groupe de formule -A²-X²-A³- qui, conjointement avec l'atome de carbone auquel A² et A³ sont attachés, définit un noyau pentagonal ou hexagonal, formule dans laquelle A² est un groupe éthylène, A³ est un groupe méthylène ou éthylène et X² est un groupe oxy, ce noyau pouvant porter un ou deux substituants choisis entre méthyle et éthyle ;
ou bien R¹ et R² forment ensemble un groupe de formule -A²-X²-A³- qui, conjointement avec l'atome d'oxygène auquel A² est attaché et avec l'atome de carbone auquel A³ est attaché, définit un noyau pentagonal, formule dans laquelle A² est un groupe méthylène, A³ est un groupe méthylène et X² est un groupe oxy, ce noyau pouvant porter un, deux ou trois substituants choisis entre méthyle et éthyle, et R³ est un groupe méthyle ou éthyle ;
ou d'un sel pharmaceutiquement acceptable de ce dérivé.

4. Procédé suivant la revendication 1 pour la production d'un dérivé hétérocyclique bicyclique de formule I dans laquelle Q est un groupe 1,3,3-triméthyl-2-oxoindoline-5-yle, 1,3-diméthyl-2-oxo-2,3-dihydrobenzimidazole-5-yle ou 3-méthyl-2-oxo-2,3-dihydrobenzothiazole-6-yle ;
A¹ est une liaison directe à X¹ ;
X¹ est un groupe thio ;
Ar est un groupe 1,3-phénylène ou 5-fluoro-1,3-phénylène ;
R¹ est un groupe méthyle ; et
R² et R³ forment ensemble un groupe de formule -A²-X²-A³- qui, conjointement avec l'atome de carbone auquel A² et A³ sont attachés, définit un noyau hexagonal, formule dans laquelle A² et A³ représentent un groupe éthylène et X² est un groupe oxy, ce noyau pouvant porter un substituant méthyle ou éthyle en alpha par rapport à X² ;
ou d'un sel pharmaceutiquement acceptable de ce dérivé.

5. Procédé suivant la revendication 2, pour la production d'un dérivé hétérocyclique bicyclique de formule I dans laquelle Q est un groupe 1,3,3-triméthyl-2-oxoindoline-5-yle,1,3-diméthyl-2-oxo-2,3-dihydrobenzimidazole-5-yle, 3-méthyl-2-oxo-2,3-dihydrobenzothiazole-6-yle ou 3,3-difluoro-1-méthyl-2-oxoindoline-5-yle ;
A¹ est une liaison directe à X¹ ;
X¹ est un groupe thio ;
Ar est un groupe 1,3-phénylène ou 5-fluoro-1,3-phénylène ;
R¹ est un groupe méthyle ; et
R² et R³ forment ensemble un groupe de formule -A²-X²-A³- qui, conjointement avec l'atome de carbone auquel A² et A³ sont attachés, définit un noyau hexagonal, formule dans laquelle A² et A³ représentent un groupe éthylène et X² est un groupe oxy, et ce noyau pouvant porter un substituant méthyle ou éthyle en alpha par rapport à X² ;
ou d'un sel pharmaceutiquement acceptable de ce dérivé.

6. Procédé de préparation d'une composition pharmaceutique, caractérisé par la mise en association d'un dérivé hétérocyclique bicyclique de formule I ou d'un sel pharmaceutiquement acceptable de ce dérivé suivant l'une quelconque des revendications 1 à 5 et d'un diluant ou support acceptable du point de vue pharmaceutique.
